(19) Europäisches Patentamt
European Patent Office
Office européen des brevets

(11) **EP 3 556 746 A1**

(12) **EUROPEAN PATENT APPLICATION**

(43) Date of publication:
**23.10.2019 Bulletin 2019/43**

(21) Application number: **18168029.9**

(22) Date of filing: **18.04.2018**

(51) Int Cl.:
*C07D 221/18* (2006.01) *C07D 401/10* (2006.01)
*C07D 239/26* (2006.01) *C07D 405/10* (2006.01)
*C07D 251/24* (2006.01) *H01L 51/00* (2006.01)
*H01L 51/50* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **SCHULZE, Benjamin**
  **01099 Dresden (DE)**
- **CARDINALI, Francois**
  **01099 Dresden (DE)**
- **SCHOLZ, Johannes**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

# (54) COMPOUND, METHOD FOR PREPARING THE SAME, ORGANIC SEMICONDUCTING LAYER, ORGANIC ELECTRONIC DEVICE, DISPLAY AND LIGHTING DEVICE COMPRISING THE SAME

(57) The present invention relates to a compound of Formula (I)

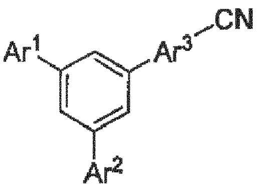

(I)

wherein $Ar^1$ is selected from substituted or unsubstituted $C_2$ to $C_{36}$ heteroaryl and the group $Ar^1$ is bound to the remaining structure represented by Formula (I) via one of its carbon atoms;
$Ar^2$ is selected from the group consisting of substituted or unsubstituted $C_6$ to $C_{36}$ aryl and substituted or unsubstituted $C_4$ to $C_{36}$ heteroaryl comprising at least one heteroatom selected from O, S and Se, wherein the heteroaryl group comprising at least one heteroatom selected from O, S and Se is bound to the remaining structure represented by Formula (I) via one of its carbon atoms;
$Ar^3$ is selected from the group consisting of substituted or unsubstituted $C_6$ to $C_{18}$ arylene and substituted or unsubstituted $C_3$ to $C_{18}$ heteroarylene, wherein it is provided that the group $Ar^3$ comprises not more than three fused rings;
wherein, in case that one or more of $Ar^1$, $Ar^2$ and $Ar^3$ is substituted, the substituents are each independently selected from the group consisting of D, F, $C_1$ to $C_{20}$ linear alkyl, $C_3$ to $C_{20}$ branched alkyl, $C_3$ to $C_{20}$ cyclic alkyl, $C_1$ to $C_{20}$ linear alkoxy, $C_3$ to $C_{20}$ branched alkoxy, linear fluorinated $C_1$ to $C_{12}$ alkyl, linear fluorinated $C_1$ to $C_{12}$ alkoxy, $C_3$ to $C_{12}$ branched fluorinated cyclic alkyl, $C_3$ to $C_{12}$ fluorinated cyclic alkyl, $C_3$ to $C_{12}$ fluorinated cyclic alkoxy, CN, RCN, $C_6$ to $C_{20}$ aryl, C2 to $C_{20}$ heteroaryl, OR, SR, (C=O)R, (C=O)$NR_2$, $SiR_3$, (S=O)R, $(S=O)_2R$, $(P=O)R_2$; wherein each R is independently selected from $C_1$ to $C_{20}$ linear alkyl, $C_1$ to $C_{20}$ alkoxy, $C_1$ to $C_{20}$ thioalkyl, $C_3$ to $C_{20}$ branched alkyl, $C_3$ to $C_{20}$ cyclic alkyl, $C_3$ to $C_{20}$ branched alkoxy, $C_3$ to $C_{20}$ cyclic alkoxy, $C_3$ to $C_{20}$ branched thioalkyl, $C_3$ to $C_{20}$ cyclic thioalkyl, $C_6$ to $C_{20}$ aryl and $C_2$ to $C_{20}$ heteroaryl; and
wherein $Ar^1$ and $Ar^2$ are selected differently from each other,
a method for preparing the same, an organic semiconducting layer comprising the same, an organic electronic device comprising the same and a display device or a lighting device comprising the organic electronic device.

EP 3 556 746 A1

## Description

The present invention relates to a compound and an organic semiconducting layer comprising the same. The present invention relates further to a method for preparing the inventive compound. The invention further relates to an organic electronic device comprising the organic semiconducting layer. Furthermore, the invention relates to a display device or a lighting device comprising the organic electronic device.

BACKGROUND ART

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. In this regard, the HTL, the EML, and the ETL are thin films formed from organic and / or organometallic compounds.

When a voltage is applied to the anode and the cathode, holes injected from the anode electrode move to the EML, via the HTL, and electrons injected from the cathode electrode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency.

Triazine compounds with aryl groups are used in organic electronics applications, especially as electron transport materials. Respective compounds often have low dipole moments. Without being bound by theory, a dipole moment of at least 2 Debye may be beneficial for device performance, in particular when the organic semiconducting layer further comprises a metal, metal salt or an alkali or alkaline earth metal complex.

Triazine compounds with heteroaryl groups, for example pyridyl groups, may have a dipole moment of at least 2 Debye. However, respective compounds may have low melting points (mp) and/or glass transition temperature (Tg). Low mp and/or Tg compounds are not preferred as materials in organic electronics because of the detrimental effect of the low mp/Tg on the durability and performance of devices comprising such compounds. Furthermore, there is still a need to improve the electronic properties of respective compounds for use in organic electronic devices, in particular to provide compounds having a high dipole moment, higher melting point and/or improved glass transition temperature and electronic properties suitable for organic electronic devices.

It is therefore an object of the present invention to provide novel organic electronic devices and compounds for use therein overcoming drawbacks of the prior art, in particular to provide novel compounds having better glass transition temperatures, improved melting points, increased dipole moments and/or electronic properties which may be suitable to improve the performance of organic electronic devices, in particular when used in an electron transport layer.

DESCRIPTION OF THE INVENTION

The above object is achieved by a compound of the Formula (I)

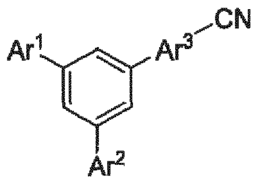

(I)

wherein $Ar^1$ is selected from substituted or unsubstituted $C_2$ to $C_{36}$ heteroaryl and the group $Ar^1$ is bound to the remaining structure represented by Formula (I) via one of its carbon atoms;

$Ar^2$ is selected from the group consisting of substituted or unsubstituted $C_6$ to $C_{36}$ aryl, alternatively $C_{10}$ to $C_{24}$ aryl, and substituted or unsubstituted $C_4$ to $C_{36}$ heteroaryl comprising at least one heteroatom selected from O, S and Se, wherein the heteroaryl group comprising at least one heteroatom selected from O, S and Se is bound to the remaining structure represented by Formula (I) via one of its carbon atoms;

$Ar^3$ is selected from the group consisting of substituted or unsubstituted $C_6$ to $C_{18}$ arylene and substituted or unsubstituted $C_3$ to $C_{18}$ heteroarylene, wherein it is provided that the group $Ar^3$ comprises not more than three fused rings; wherein, in case that one or more of $Ar^1$, $Ar^2$ and $Ar^3$ is substituted, the substituents are each independently selected from the group consisting of D, F, $C_1$ to $C_{20}$ linear alkyl, $C_3$ to $C_{20}$ branched alkyl, $C_3$ to $C_{20}$ cyclic alkyl,

$C_1$ to $C_{20}$ linear alkoxy, $C_3$ to $C_{20}$ branched alkoxy, linear fluorinated $C_1$ to $C_{12}$ alkyl, linear fluorinated $C_1$ to $C_{12}$ alkoxy, $C_3$ to $C_{12}$ branched fluorinated cyclic alkyl, $C_3$ to $C_{12}$ fluorinated cyclic alkyl, $C_3$ to $C_{12}$ fluorinated cyclic alkoxy, CN, RCN, $C_6$ to $C_{20}$ aryl, $C_2$ to $C_{20}$ heteroaryl, OR, SR, (C=O)R, (C=O)$NR_a$, $SiR_3$, (S=O)R, $(S=O)_2R$, $(P=O)R_2$; wherein each R is independently selected from $C_1$ to $C_{20}$ linear alkyl, $C_1$ to $C_{20}$ alkoxy, $C_1$ to $C_{20}$ thioalkyl, $C_3$ to $C_{20}$ branched alkyl, $C_3$ to $C_{20}$ cyclic alkyl, $C_3$ to $C_{20}$ branched alkoxy, $C_3$ to $C_{20}$ cyclic alkoxy, $C_3$ to $C_{20}$ branched thioalkyl, $C_3$ to $C_{20}$ cyclic thioalkyl, $C_6$ to $C_{20}$ aryl and $C_2$ to $C_{20}$ heteroaryl; and

wherein $Ar^1$ and Ar2 are selected differently from each other.

It has surprisingly been found by the inventors that compounds of Formula (I) have improved melting points, increased glass transition temperatures and increase dipole moments compared to compounds known in the art. Furthermore, it has surprisingly been found that the compounds are suitable to improve the performance of organic electronic devices, such as OLEDs, when being comprised in organic semiconducting layers of such devices.

In the inventive compound, $Ar^1$ may be selected from $C_3$ to $C_{26}$ heteroaryl, alternatively $C_3$ to $C_{24}$ heteroaryl. In this way, fine tuning of the electronic structure of the compound of Formula (I) can be achieved to further improve the usability thereof in organic semiconducting layers of organic electronic devices in particular in electron transport layers.

In the inventive compound, $Ar^1$ may comprise at least one N atom; alternatively $Ar^1$ may comprise between two and three N atoms. In this way, fine tuning of the electronic structure of the compound of Formula (I) can be achieved to further improve the usability thereof in organic semiconducting layers of organic electronic devices in particular in electron transport layers.

In the inventive compound, $Ar^1$ may comprise a pyridine, triazine, pyrimidine, acridine, benzoacridine, dibenzoacridine, benzimidazole, quinazoline, quinoxaline or benzoquinazoline group which is bound to the remaining structure represented by Formula (I) via one of the carbon atoms thereof. In this way, fine tuning of the electronic structure of the compound of Formula (I) can be achieved to further improve the usability thereof in organic semiconducting layers of organic electronic devices in particular in electron transport layers.

$Ar^1$ may be free of carbazole and/or indolocarbazole groups.

$Ar^1$ may be selected from an acridine, benzoacridine and/or dibenzoacridine group; alternatively $Ar^1$ may be selected from a benzoacridine and/or dibenzoacridine group.

$Ar^1$ may be selected from a triazine and/or pyrimidine group, preferably a triazine group.

In the inventive compound, the heteroaryl group may comprise at least one heteroatom selected from O, S and Se and does not comprise any other heteroatoms. In this way, fine tuning of the electronic structure of the compound of Formula (I) can be achieved to further improve the usability thereof in organic semiconducting layers of organic electronic devices in particular in electron transport layers.

In the inventive compound, the heteroaryl group may comprise at least one heteroatom selected from O, S and Se and comprise only one heteroatom. In this way, fine tuning of the electronic structure of the compound of Formula (I) can be achieved to further improve the usability thereof in organic semiconducting layers of organic electronic devices in particular in electron transport layers.

In the inventive compound, $Ar^2$ may comprise at least two fused rings. In this way, fine tuning of the electronic structure of the compound of Formula (I) can be achieved to further improve the usability thereof in organic semiconducting layers of organic electronic devices in particular in electron transport layers.

In the inventive compound, $Ar^2$ may comprise two to three fused rings. In this way, fine tuning of the electronic structure of the compound of Formula (I) can be achieved to further improve the usability thereof in organic semiconducting layers of organic electronic devices in particular in electron transport layers.

In the inventive compound, $Ar^2$ may comprise an ethylene group substituted with four groups independently selected from phenyl and pyridyl. In this way, fine tuning of the electronic structure of the compound of Formula (I) can be achieved to further improve the usability thereof in organic semiconducting layers of organic electronic devices in particular in electron transport layers.

In the inventive compound, $Ar^2$ may be selected from the group consisting of substituted or unsubstituted $C_{10}$ to $C_{36}$ aryl and substituted or unsubstituted $C_6$ to $C_{36}$ heteroaryl comprising at least one heteroaryl atom selected from O, S, Se; alternatively from the group consisting of substituted or unsubstituted $C_{10}$ to $C_{24}$ aryl and substituted or unsubstituted $C_8$ to $C_{24}$ heteroaryl comprising at least one heteroatom selected from O, S, Se; alternatively from the group consisting of substituted or unsubstituted $C_{10}$ to $C_{16}$ aryl and substituted or unsubstituted $C_8$ to $C_{16}$ heteroaryl comprising at least one heteroatom selected from O, S, and Se. In this way, fine tuning of the electronic structure of the compound of Formula (I) can be achieved to further improve the usability thereof in organic semiconducting layers of organic electronic devices in particular in electron transport layers.

In the inventive compound, $Ar^2$ may be selected from groups having the following formulas a to k

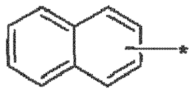

a

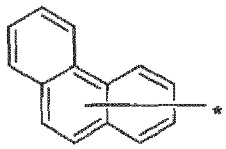

b

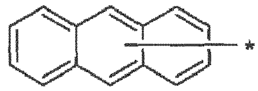

c

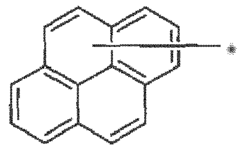

d

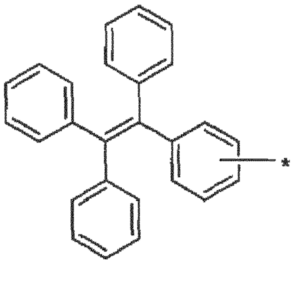

e

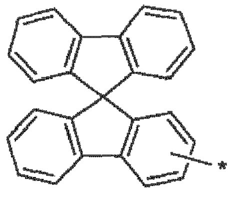

f

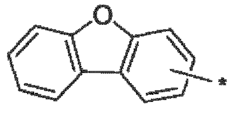

g

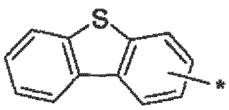

h

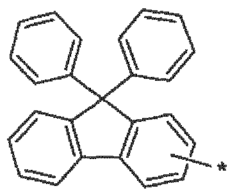

i

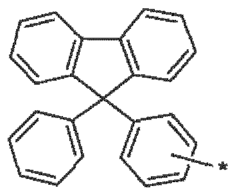

j

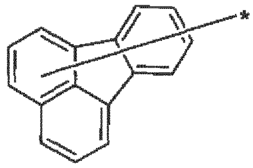

k,

wherein the asterisk symbol "*" in the above formulas represents the position of binding of the respective group to the remaining structure represented by Formula (I). In this way, fine tuning of the melting point and or glass transition temperature of the compound of Formula (I) can be achieved to further improve the usability thereof in organic semiconducting layers of organic electronic devices in particular in electron transport layers.

In the inventive compound, $Ar^3$ may be selected from the group consisting of substituted or unsubstituted $C_6$ to $C_{12}$ arylene and substituted or unsubstituted $C_5$ to $C_{16}$ heteroarylene, alternatively from substituted or unsubstituted $C_6$ to $C_{12}$ arylene. In this way, fine tuning of the electronic structure of the compound of Formula (I) can be achieved to further improve the usability thereof in organic semiconducting layers of organic electronic devices in particular in electron transport layers.

Ar3 may comprise two fused rings, alternatively zero fused rings.

In case that $Ar^3$ may be substituted, the one or more substituents may independently selected from the group consisting of D, F, $CF_3$ and $C_1$ to $C_{12}$ alkyl. In this way, fine tuning of the electronic structure of the compound of Formula (I) can be achieved to further improve the usability thereof in organic semiconducting layers of organic electronic devices in particular in electron transport layers.

$Ar^1$, $Ar^2$ and $Ar^3$ may be unsubstituted. Thereby, particularly beneficial performance in organic electronic devices may be obtained, when the compound of Formula (I) is evaporated and deposited on the substrate in high vacuum.

In the inventive compound, $Ar^1$ may be selected from formulas 1 to dd

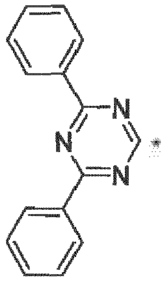

l

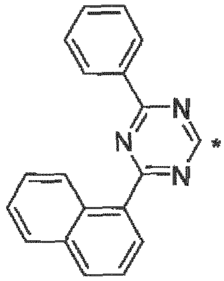

m

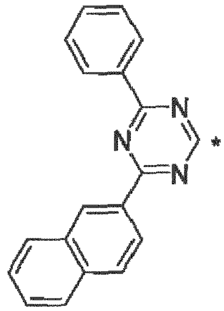

n

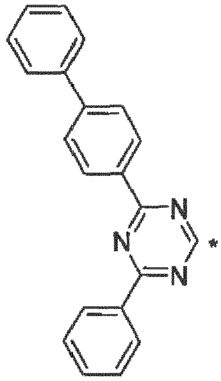

o

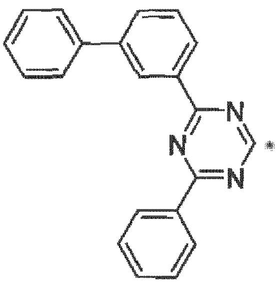

p

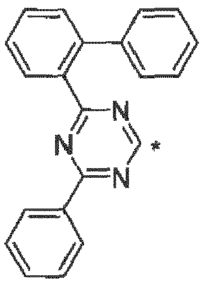

q

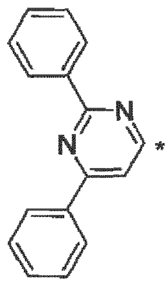

r

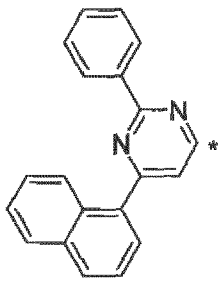

s

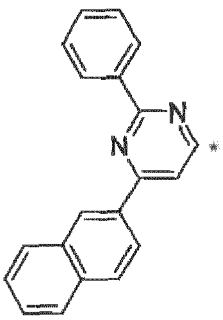

t

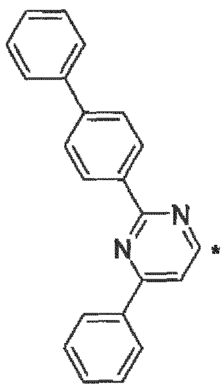

u

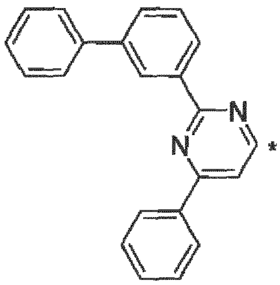

v

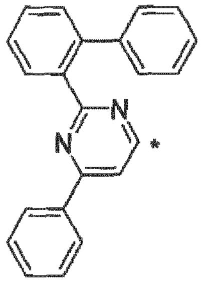 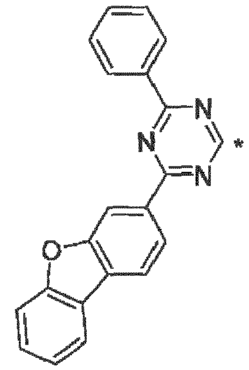 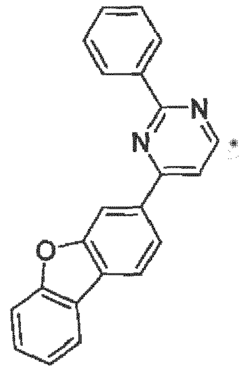 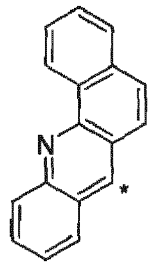

w x y z

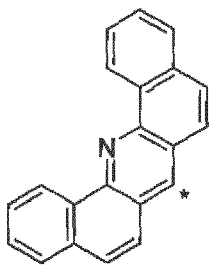 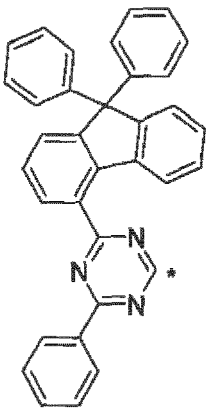 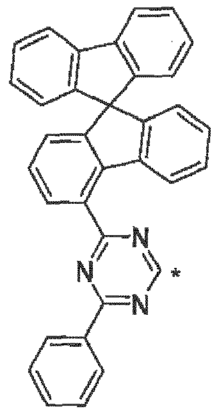 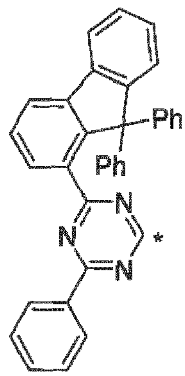

aa bb cc dd,

wherein the asterisk symbol "*" in the above formulas represents the position of binding of the respective group to the remaining structure represented by Formula (I). In this way, fine tuning of the electronic structure of the compound of Formula (I) can be achieved to further improve the usability thereof in organic semiconducting layers of organic electronic devices in particular in electron transport layers.

The compound may be selected from the following structures A-1 to A-18

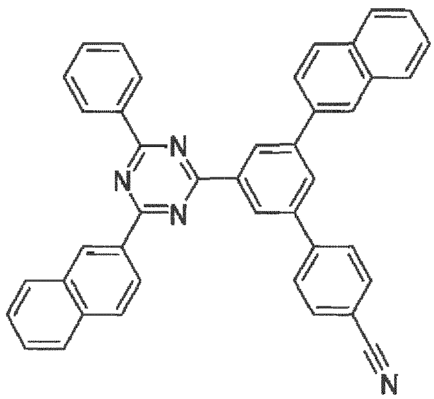

A-1

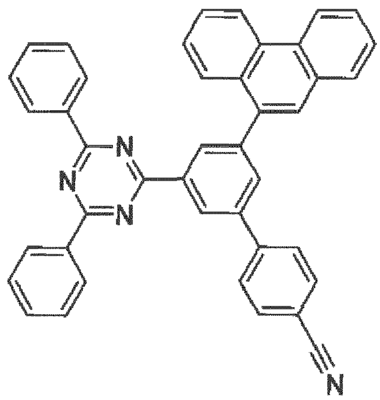

A-2

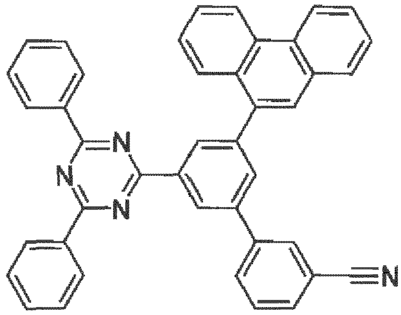
A-3

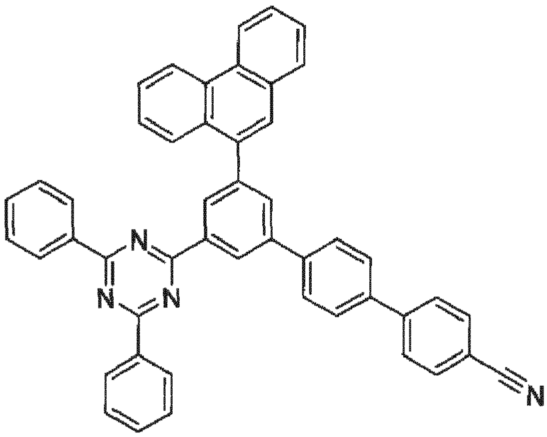
A-4

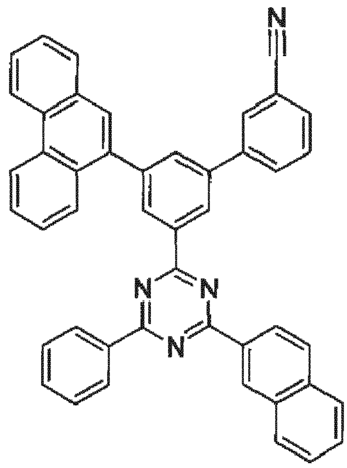
A-5

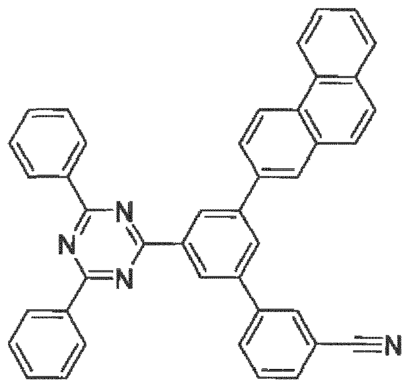
A-6

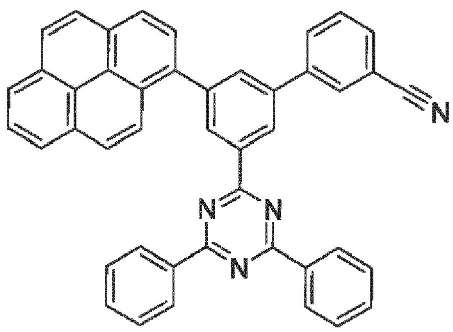
A-7

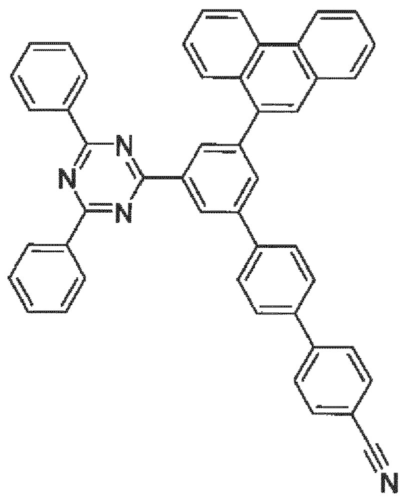
A-8

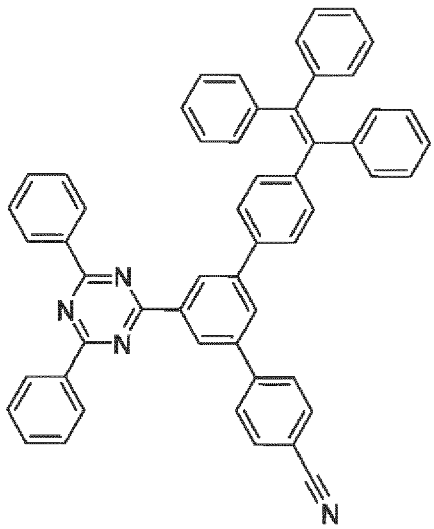

A-9

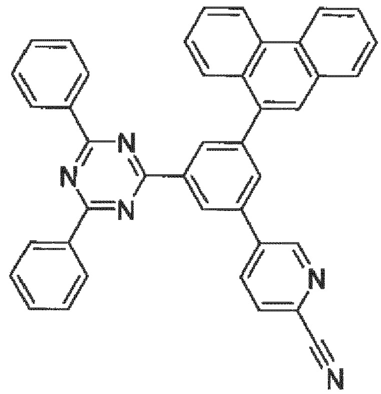

A-10

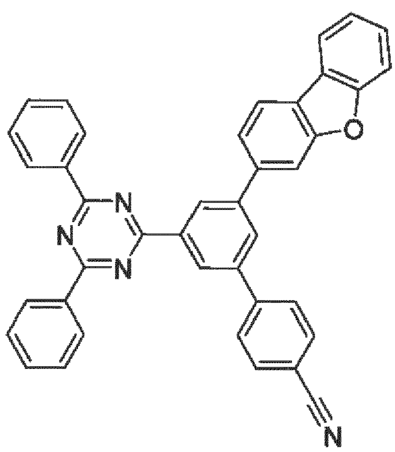

A-11

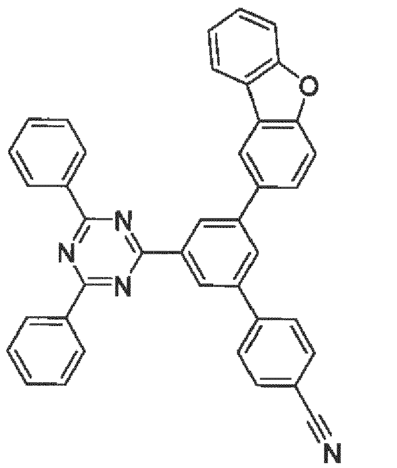

A-12

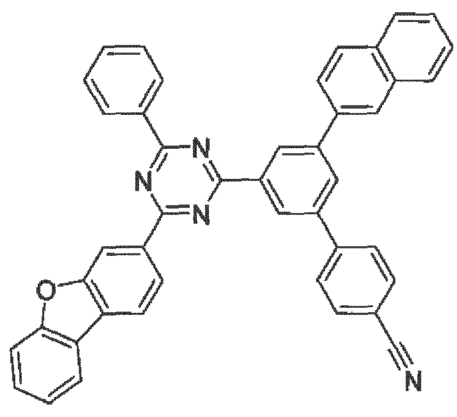

A-13

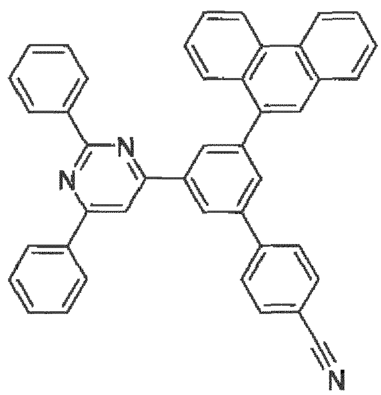

A-14

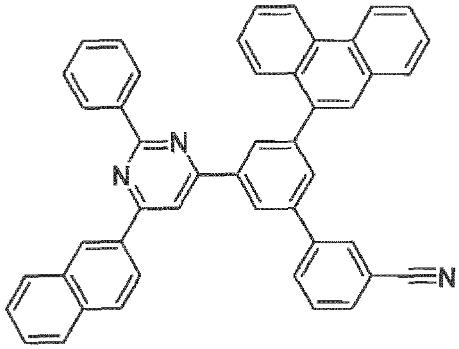

A-15

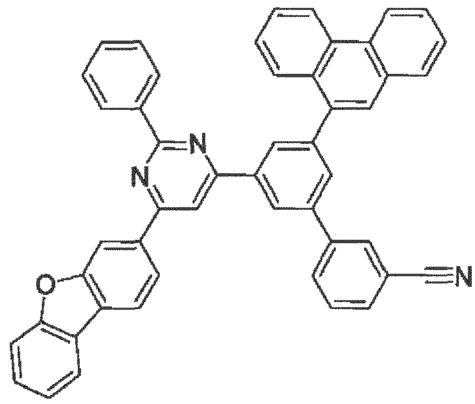

A-16

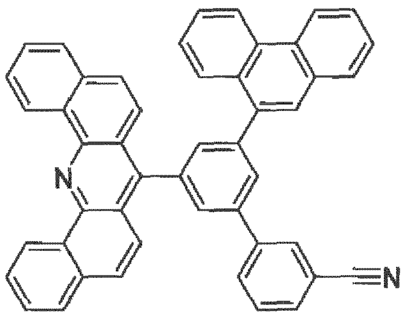

A-17

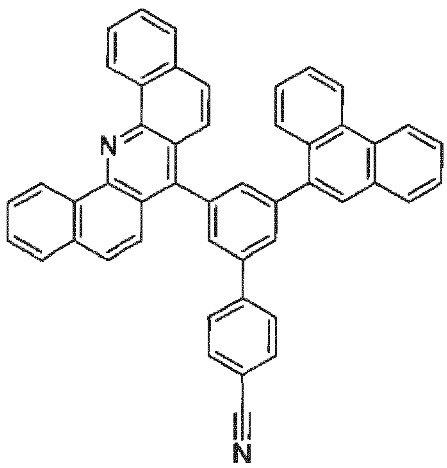

A-18

In this way, fine tuning of the electronic structure of the compound of Formula (I) can be achieved to further improve the usability thereof in organic semiconducting layers of organic electronic devices in particular in electron transport layers.

It has surprisingly been found by the inventors that compounds of Formula (I) have improved melting points, increased glass transition temperatures and increase dipole moments compared to compounds known in the art. Furthermore, it has surprisingly been found that the compounds are suitable to improve the performance of organic electronic devices, such as OLEDs, when being comprised in organic semiconducting layers of such devices.

Furthermore, the object is achieved by an organic semiconducting layer comprising at least one inventive compound of Formula (I).

The organic semiconducting layer maybe non-emissive.

The organic semiconducting layer may further comprise a metal, a metal salt or an alkali or alkaline earth metal complex, alternatively an organic alkali or alkaline earth metal complex; alternatively 8-hydroxyquinolinolato-lithium or an alkali borate.

The organic semiconducting layer may consist of a compound of Formula (I).

In the organic semiconducting layer comprising or consisting of the compound of Formula (I) may be non-emissive.

The organic semiconducting layer may further comprise a metal, a metal salt or an alkali or alkaline earth metal complex; alternatively an organic alkali or alkaline earth metal complex; alternatively 8-hydroxyquinolinolato lithium or alkali borate.

The object is further achieved by an organic electronic device comprising the inventive organic semiconducting layer.

The organic electronic device comprising the inventive organic semiconducting layer may further comprise an anode, a cathode and an emission layer.

In the organic electronic device, the organic semiconducting layer comprising compound of Formula (I) may

be arranged between the anode and the cathode. Preferably, the organic semiconducting layer may be arranged between the emission layer and the cathode. When the organic semiconducting layer is arranged in this way, it may function as electron transport layer.

In the organic electronic device, the organic semiconducting layer may be in direct contact with the emission layer.

The organic electronic device may comprise further comprise a hole blocking layer, wherein the hole blocking layer is in direct contact with the emission layer and the organic semiconductor layer comprising the compound of Formula (I) is arranged between the hole blocking layer and the cathode.

The organic electronic device may further comprise a hole blocking layer wherein the organic semiconducting layer may be arranged between the hole blocking layer and the cathode and may be in direct contact with the hole blocking layer and the cathode.

In the organic electronic device, the organic semiconducting layer may comprise a metal, a metal salt or an alkali or alkaline earth metal complex, alternatively an organic alkali or alkaline earth metal complex, alternatively 8-hydroxyquinolinolato-lithium or an alkali borate.

The object is further achieved by a display device comprising the inventive organic electronic device.

The object is further achieved by a lighting device comprising the organic electronic device.

The object is further achieved by a process for preparing a compound of Formula (I) comprising the steps of

(a) providing a compound of Formula 2 or 3

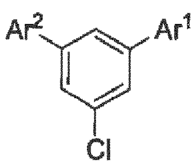

Formula 2

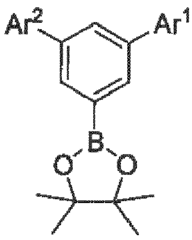

Formula 3;

(b) reacting the compound of Formula 2 with a compound having the formula NC-$Ar^3$-$B(OR)_2$, wherein R is selected from H, $C_1$ to $C_4$ alkyl or

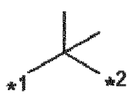

,

wherein the asterisk symbol "*1" in the above formula represents the position of binding of the respective group to the O and the asterisk symbol "*2" in above formula represents the position of binding of the respective group to the other R;

or reacting the compound of Formula 3 with a compound having the formula NC-$Ar^3$-Y, wherein Y is selected from the group consisting of I, Br, Cl and OTf.

In the process, the step (a) of providing the compound of Formula 2 may comprise the steps of

(a') providing a compound of Formula 4 or 5

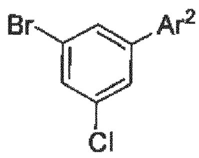
Formula 4

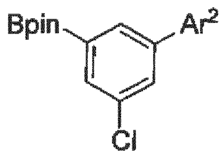
Formula 5;

(b') reacting the compound of Formula 4 with a compound having the formula $Ar^1$-$B(OR)_2$ or reacting the compound of Formula 5 with $Ar^1$-Y.

The process may further comprise a step (a") before the step (a') of reacting a compound of Formula 6

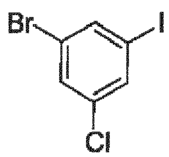
Formula 6

with $Ar^2$-$B(OR)_2$ to achieve the compound of Formula 4.

The process may comprise the following process steps

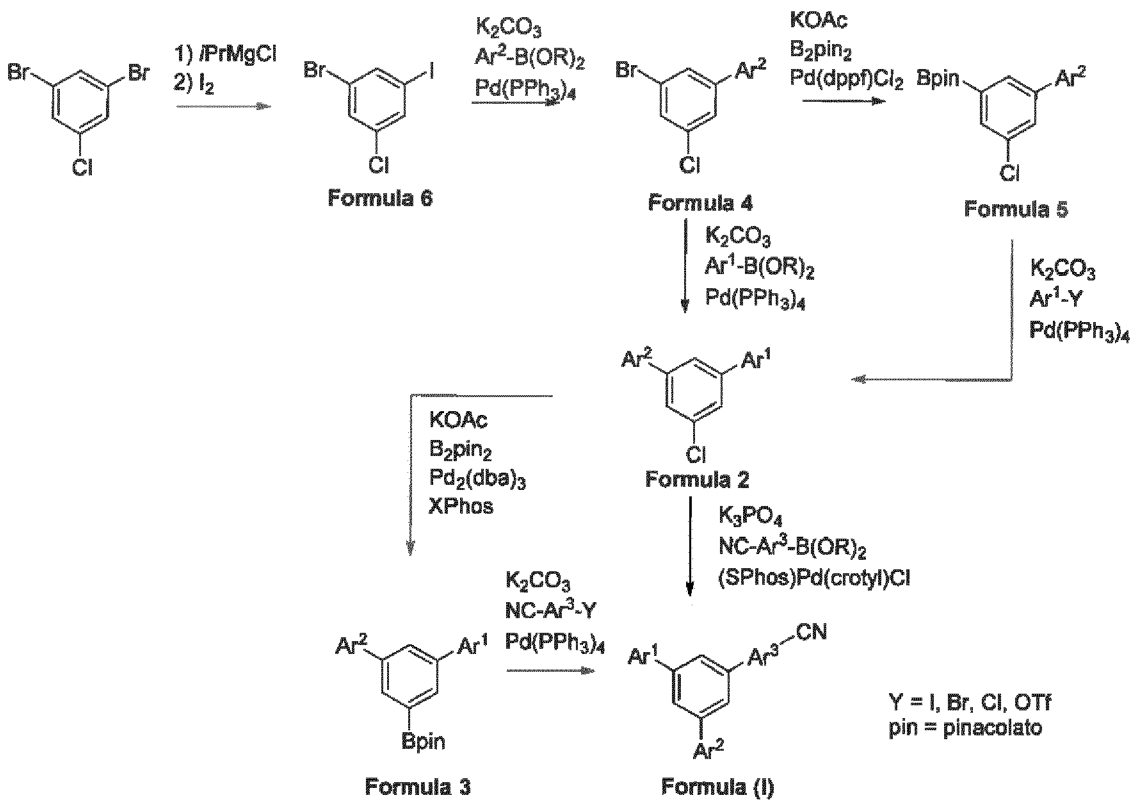


Finally, the object is achieved by a process for preparing a compound of Formula (I) comprising the steps of

(a) providing a compound of Formula 7

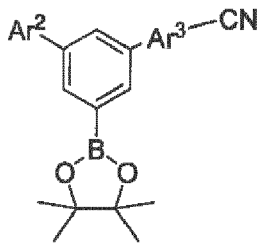

Formula 7;

(b) reacting the compound of Formula 7 with a compound having the formula $Ar^1$-Y, wherein Y is selected from the group consisting of I, Br, Cl and OTf.

In the process, the step (a) of providing the compound of Formula 7 may comprise the steps of

(a') providing a compound of Formula 8

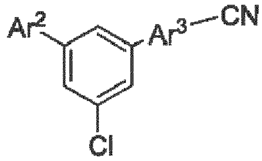

Formula 8;

(b') reacting the compound of Formula 8 with a compound having the formula $B_2pin_2$.

The process may further comprise the step (a') of providing the compound of Formula 8 may comprise the steps of

(a") providing a compound of Formula 9

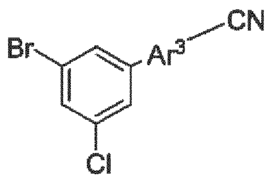

Formula 9;

(b") reacting the compound of Formula 9 with a compound having the formula $Ar^2$-$B(OR)_2$.

The process may further comprise a step (a'") before the step (a") of reacting a compound of Formula 6

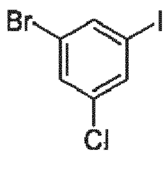

Formula 6

with $Ar^2$-$B(OR)_2$ to achieve the compound of Formula 9.

The process may comprise the following process steps

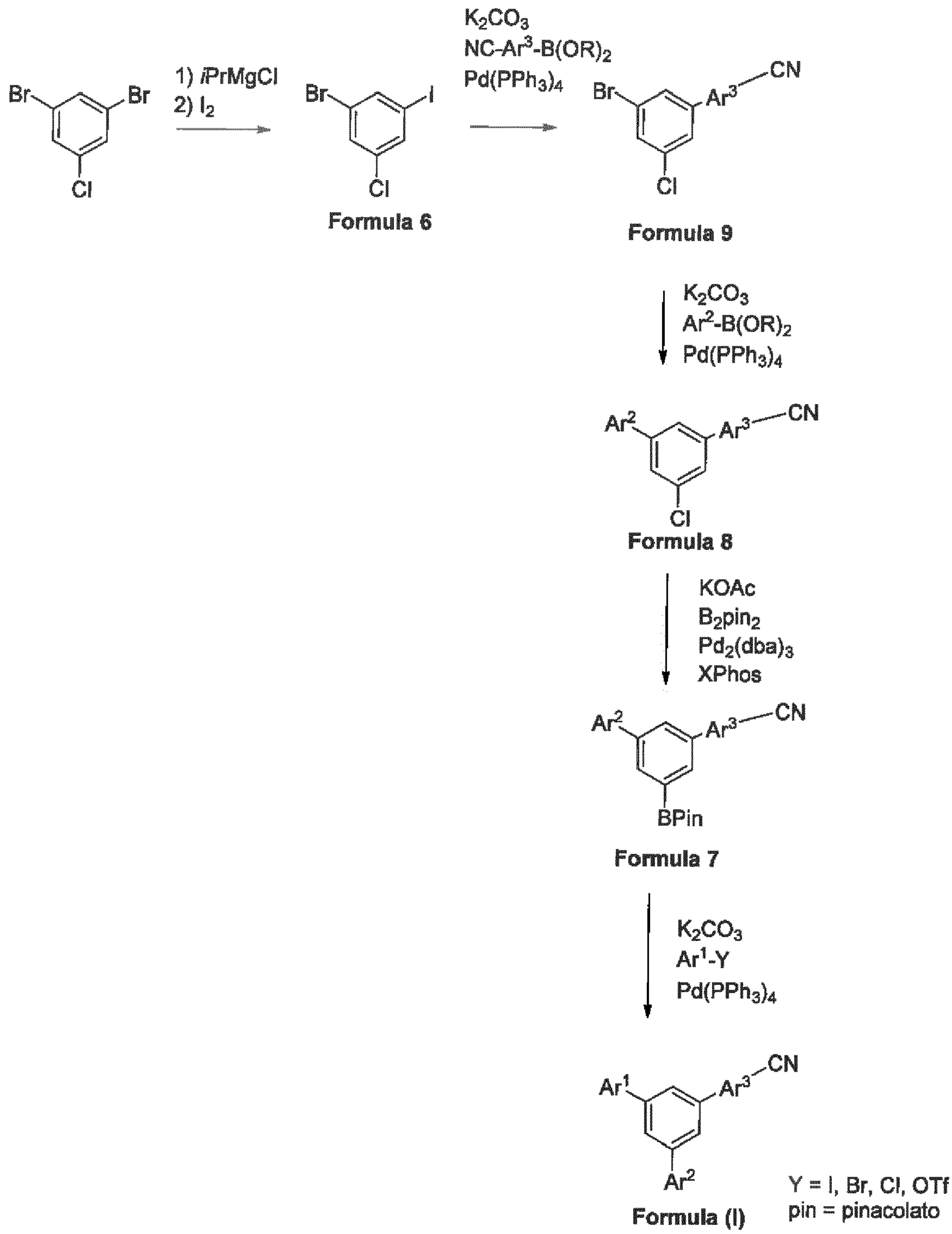

Formula 6

Formula 9

Formula 8

Formula 7

Formula (I)

In accordance with the invention, the structures shown in Table 1 below are most preferred.

Further layers

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as

organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris(3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.
When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.
If the electron blocking layer has a high triplet level, it may also be described as triplet control layer.
The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.
It may be provided that the emission layer does not comprise the compound of Formula (I).
The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl) -triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).
The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.
Examples of red emitter dopants are PtOEP, Ir(piq)3, and Btp2lr(acac), but are not limited thereto. These compounds are phosphorescent emitters, however, fluorescent red emitter dopants could also be used.
Examples of phosphorescent green emitter dopants are Ir(ppy)3 (ppy = phenylpyridine), Ir(ppy)2(acac), Ir(mpyp)3.
Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.
The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconducting layer comprising or consisting of the inventive compound represented by the general Formula (I) as defined above.
The HBL may also be named auxiliary ETL or a-ETL.
When the HBL is formed using vacuum deposition or spin coating, the conditions for deposition and coating

may be similar to those for the formation of the HIL. However, the conditions for deposition and coating may vary, according to the compound that is used to form the HBL. Any compound that is commonly used to form a HBL may be used. Examples of compounds for forming the HBL include xadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The OLED according to the present invention may comprise an electron transport layer (ETL). In accordance with the invention, the electron transport layer may be the inventive organic semiconducting layer comprising the inventive compound represented by the general Formula (I) as defined above.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (I) as defined above as the organic electron transport matrix (ETM) material. The electron transport layer may comprise, besides the compound represented by the general Formula (I), further ETM materials known in the art. Likewise, the electron transport layer may comprise as the only electron transport matrix material the compound represented by general Formula (I). In case that the inventive organic electronic device comprises more than one electron transport layers, the compound represented by the general Formula (I) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (I) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1970 371 A1 or WO 2013/079217 A1.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the organic semiconducting layer comprising the compound of Formula (I).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

*Charge generation layer/hole generating layer*

The charge generation layer (CGL) may comprise a p- type and an n-type layer. An interlayer may be arranged between the p-type layer and the n-type layer.

Typically, the charge generation layer is a pn junction joining a n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem devices, for example, in tandem OLEDs comprising, between two electrodes, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer may be arranged in direct contact to the n-type charge generation layer.

*Organic light-emitting diode (OLED)*

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconducting layer comprising a compound of Formula (I) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconducting layer comprising a compound of Formula (I) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconducting layer comprising a compound of Formula (I) , an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is

adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconducting layer according to the invention may be the electron transport layer, first electron transport layer, n-type charge generation layer and/or second electron transport layer.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

## Organic electronic device

An organic electronic device according to the invention comprises an organic semiconducting layer comprising a compound according to Formula (I).

An organic electronic device according to one embodiment may include a substrate, an anode layer, an organic semiconducting layer comprising a compound of Formula (I) and a cathode layer.

An organic electronic device according to one embodiment comprises at least one organic semiconducting layer comprising at least one compound of Formula (I), at least one anode layer, at least one cathode layer and at least one emission layer, wherein the organic semiconducting layer is preferably arranged between the emission layer and the cathode layer.

An organic light-emitting diode (OLED) according to the invention may include an anode, a hole transport layer (HTL), an emission layer (EML), an electron transport layer (ETL) comprising at least one compound of Formula (I), and a cathode, which are sequentially stacked on a substrate. In this regard, the HTL, the EML, and the ETL are thin films formed from organic compounds.

An organic electronic device according to one embodiment can be a light emitting device, thin film transistor, a battery, a display device or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release the metal, a metal salt or an alkali or alkaline earth metal complex; alternatively an organic alkali or alkaline earth metal complex; alternatively 8-hydroxyquinolinolato lithium or alkali borate;

the method comprising the steps of forming the organic semiconducting layer; whereby for an organic light-emitting diode (OLED):

- the organic semiconducting layer is formed by releasing the compound of Formula (I) according to the invention from the first deposition source and a metal, a metal salt or an alkali or alkaline earth metal complex; alternatively an organic alkali or alkaline earth metal complex; alternatively 8-hydroxyquinolinolato lithium or alkali borate, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an organic semiconducting layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconducting layer and the cathode electrode.

According to various embodiments of the present invention, the method may further include forming an electron injection layer on the organic semiconducting layer . However, according to various embodiments of the OLED of the present invention, the OLED may not comprise an electron injection layer.

According to various embodiments, the OLED may have the following layer structure, wherein the layers having the following order:
anode, hole injection layer, first hole transport layer, second hole transport layer, emission layer, optional hole blocking layer, organic semiconducting layer comprising a compound of Formula (I) according to the invention, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconducting layer comprising a compound according to Formula (I) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

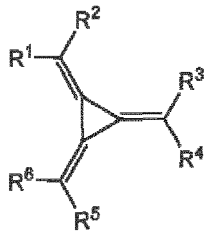 (XX) 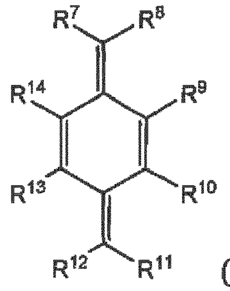 (XXIa) 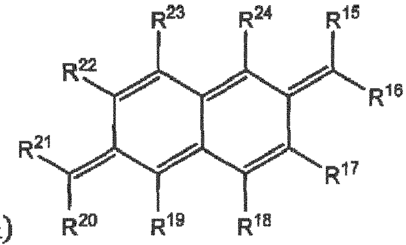 (XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Hereinafter, the embodiments are illustrated in more detail with reference to examples. However, the present disclosure is not limited to the following examples. Reference will now be made in detail to the exemplary aspects.

Details and definitions of the invention

As explained above, the inventive compound has the general Formula (I).

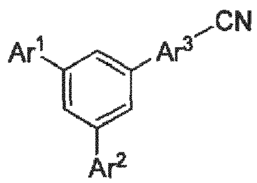 (I)

In this compound, the group $Ar^1$ is a $C_2$ to $C_{36}$ heteroaryl group. As explained below in detail, the term "heteroaryl" refers to a compound comprising, besides the carbon atoms, at least one further heteroatom, preferably selected from N, O, S, B or Si. In accordance with the invention, it is required that the group $Ar^1$ is connected with the phenylene moiety of the compound of Formula (I) via a carbon atom thereof. That is, the connection between $Ar^1$ and the remaining part of the structure of Formula (I) is not made via one of the heteroatoms. This is of particular importance if the heteroatom of the heteroaryl group $Ar^1$ is nitrogen.

Substantially the same is true with respect to the group $Ar^2$. This group may be a $C_4$ to $C_{36}$ heteroaryl group comprising at least one heteroatom selected from O, S and Si. Even if these elements are divalent, i.e. if comprised in the heteroaryl group may not be used for connecting this group $Ar^2$ to the remaining part of the structure of Formula (I), the presence of further heteroatoms, such as N, is, in principle, not excluded. In such a case, it is, however, provided that the connection between the moiety $Ar^2$ and the remaining part of the structure is via one of the carbon atoms comprised in the group $Ar^2$.

According to one embodiment of the invention, it is provided that the group $Ar^2$ comprises an ethylene group substituted with four groups independently selected from phenyl and pyridyl. This binding situation refers to the following structure:

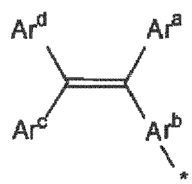

In this structure. the groups $Ar^a$ to $Ar^d$ are independently selected from phenyl and pyridyl. In this regard, it is provided that one of these groups is selected as phenylene or pyridinylene. The second binding position "*" is then used to connect the above moiety either directly to the remaining part of Formula (I) or for connecting the above moiety to further arylene group which is then connected to the remaining part of Formula (I). In the above structural formula, this phenylene or pyridinylene group is, exemplarily, the group $Ar^b$.

With respect to the inventive process OTf refers to triflate, i.e. a functional group with the formula $CF_3SO_3^-$. "Bpin" refers to boron pinacolato, i.e. a group $BO_2C_6H_{12}$. A respective group is, for example, comprised in the compound

of Formula 3. "*i*Pr" as used herein is for iso-propyl. The abbreviation "dppf" is for diphenylphosphinoferrocene. "SPhos" is for 2-dicyclohexylphosphino-2',6'-dimethoxybiphenyl. "XPhos" is for 2-dicyclohexylphosphino-2',4',6'-triisopropylbiphenyl. The abbreviation "dba" is for dibenzylidene acetone. "pin" is for pinacolato.

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl. The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused (condensed) aromatics, such as naphthalene, anthracene, phenanthracene, tetracene etc. Further encompassed are biphenyl and oligo- or polyphenyls, such as terphenyl etc.. Further encompassed shall be any further aromatic hydrocarbon substituents, such as fluorenyl etc. "Arylene" respectively "heteroarylene", referres to groups to which two further moieties are attached. In the present specification the term "aryl group" or "arylene group" may refer to a group comprising at least one hydrocarbon aromatic moiety, and all the elements of the hydrocarbon aromatic moiety may have p-orbitals which form conjugation, for example a phenyl group, a naphtyl group, an anthracenyl group, a phenanthrenyl group, a pyrenyl group, a fluorenyl group and the like. The aryl or arylene group may include a monocyclic or fused ring polycyclic (i.e., rings sharing adjacent pairs of carbon atoms) functional group.

The term "heteroaryl" as used herein refers to aryl groups in which at least one carbon atom is substituted with a heteroatom, preferably selected from N, O, S, B or Si.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably a heteroarylene ring may comprise at least 1 to 3 heteroatoms individually selected from N, S and/or O.

The term "heteroaryl" as used herewith shall encompass pyridine, quinoline, quinazoline, pyridine, triazine, benzimidazole, benzothiazole, benzo[4,5]thieno[3,2-d]pyrimidine, carbazole, xanthene, phenoxazine, benzoacridine, dibenzoacridine and the like.

"Fused rings" are those two rings sharing two adjacent atoms with each other. In other words, the rings share one covalent bond, i.e. the so-called bridge head atoms are directly connected.

In the present specification, the single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

With respect to the inventive organic semiconductive layer as well as with respect to the inventive compound, the compounds mentioned in the experimental part are most preferred.

The inventive organic electronic device may be an organic electroluminescent device (OLED) an organic photovoltaic device (OPV), a lighting device, or an organic field-effect transistor (OFET). A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention may comprise more than one emission layer, preferably two or three emission layers. An OLED comprising more than one emission layer is also described as a tandem OLED or stacked OLED.

The organic electroluminescent device (OLED) may be a bottom- or top-emission device.

Another aspect is directed to a device comprising at least one organic electroluminescent device (OLED). A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula [AS1]

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula I is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

All numeric values are herein assumed to be modified by the term "about", whether or not explicitly indicated. As used herein, the term "about" refers to variation in the numerical quantity that can occur.

Whether or not modified by the term "about" the claims include equivalents to the quantities.

The anode electrode and cathode electrode may be described as anode electrode / cathode electrode or anode electrode / cathode electrode or anode electrode layer / cathode electrode layer.

[AS2] Room temperature, also named ambient temperature, is 23°C.

## BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention;

FIG. 2 is a schematic sectional view of an OLED, according to an exemplary embodiment of the present invention.

FIG. 3 is a schematic sectional view of a tandem OLED comprising a charge generation layer, according to an exemplary embodiment of the present invention.

DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155.

Referring to Fig. 2, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconducting layer comprising a compound of Formula (I) may be an ETL.

Fig. 3 is a schematic sectional view of a tandem OLED 200, according to another exemplary embodiment of the present invention. Fig. 3 differs from Fig. 2 in that the OLED 100 of Fig. 3 further comprises a charge generation layer (CGL) and a second emission layer (151).

Referring to Fig. 3, the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconducting layer comprising a compound of Formula (I) may be a first ETL, n-type CGL and/or second ETL.

While not shown in Fig. 1, Fig. 2 and Fig. 3, a sealing layer may further be formed on the cathode electrodes 190, in order to seal the OLEDs 100 and 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

## Experimental data

Preparation of compounds of Formula (I)

Compounds of Formula (I) may be synthesized as described below.

Preparation of compound of Formula 6

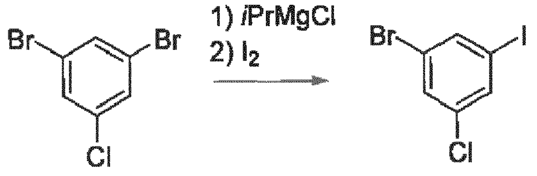


**Formula 6**

Under nitrogen atmosphere, iPrMgCl (30 mL, 39 mmol, 1.05eq.) was added to a solution of 1 eq. 1,3-dibromo-5-chlorbenzene (10 g, 37 mmol) cooled at -25 °C. The reaction was let get back to room temperature and stirred for 16 h. Elementary Iodine was then added in portions (10 g, 40,7 mmol, 1.1 eq.) under positive pressure of nitrogen. The reaction mixture was stirred 17 h at room temperature. Neutralization of the excess iodine was performed via addition of 20 mL sat. Na2S2O3 aqueous solution, then after addition of another 30 mL of water, THF was evaporated. Extraction with 250 mL Dichloromethane followed. After washing the organic layer with brine, filtering off insolubles, drying the organic layer on MgSO4, addition of 100mL of cyclohexane and evaporation of the dichloromethane, was obtained 9.6 g (82 %) solid. GC-MS 94.4 % m/z 316,318 [M]+

Example of reaction conditions which can be suitably used for preparation of compound of Formula 4 from compound of Formula 6. compound of Formula 2 from compound of Formula 4. compound of Formula (I) from compound of Formula 3. compound of Formula 9 from compound of Formula 6, compound of Formula 8 from compound of Formula 9 and compound of Formula (I) from compound of Formula 7

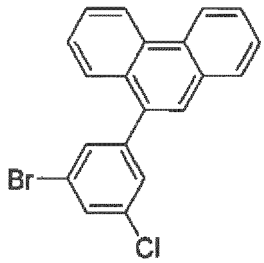

Potassium carbonate (as 2 M aqueous solution, 2 eq.) was degassed with nitrogen for 30 min. Dioxane (976 ml) was also degassed with nitrogen for 30 min. The THF flask was then charged with corresponding 9-phenanthreneboronic acid (10 mmol, 1.32 eq.), previously synthesized formula 6 intermediate (78 g, 1 eq.) and tetrakis(triphenylphosphin)palladium(0) (0.035 eq.) under a positive nitrogen pressure. The degassed potassium carbonate solution was added, nitrogen purged reflux condenser was attached to the flask and a reaction mixture heated to 65 °C with stirring for 48 h. The mixture was allowed to cool down to the room temperature, dioxane was evaporated. Extraction with 1.75 L chloroform was performed. After washing the organic layer with 5 x 250 mL water, it was dried over MgSO4 and filtered over silicagel layer. Evaporation almost to dryness followed by hexane addition provided a precipitate that was still colored. Further washing with Na2S2O3 sat. aqueous solution afforded 49.2g (54%) product, m/z 366,368 [M]+

The following compound were prepared using similar reaction conditions

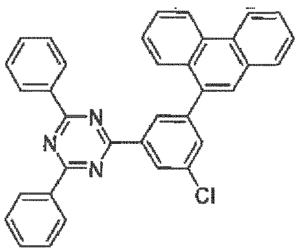

Starting from Chloro-4,6-diphenyl-1,3,5-triazine (1.05 eq.) and 2-{3-chloro-5-(phenanthren-9-yl)phenyl)-4,4,5,5-tetramethyl-1,3,2-dioxaborolane (36.3 g, 1 eq.), obtained 33.6g (74 %) product, m/z 520 $[M+H]^+$

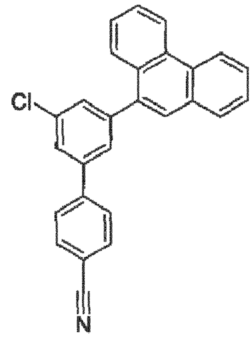

Starting from 9-phenanthreneboronic acid (1 eq.) and 3'-bromo-5'-chloro-[1,1'-biphenyl]-4-carbonitrile (12,8g, 1eq.), obtained 6,9g (40%) product, m/z 389 [M]+

Example of reaction conditions which can be suitably used for preparation of compound of Formula 5 from compound of Formula 4

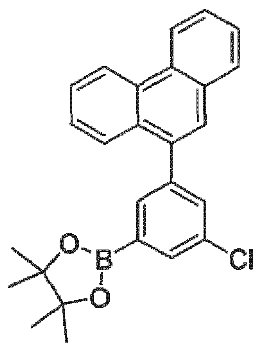

Under nitrogen, a flask was charged with 49 g from previous intermediate, in 1 L dry dioxane. Under positive pressure of nitrogen were added 1.1 eq. bis(pinacolato)diboron, 6 mol.-% of catalyst Pd(dppf)Cl2 and finally 2.5 eq. of potassium acetate. The reaction mixture was heated to 80 °C with stirring for 21 h. The mixture was allowed to cool down to the room temperature and dioxane was removed under reduced pressure. Extraction with 0,7 L chloroform was performed. After washing the organic layer with 3 x 250 mL water, it was dried over MgSO4 and filtered over silicagel layer. Evaporation almost to dryness followed by addition of hexane allowed to filter off insoluble impurities. Mother liquor was then evaporated and treated by slurry wash in acetonitrile followed by dissolution in hot toluene and filtration over silicagel layer. Precipitation with acetonitrile afforded 36,5g (66%) product, m/z 414 $[M]^+$

Example of reaction conditions which can be suitably used for preparation of compound of Formula 7 from compound of Formula 8

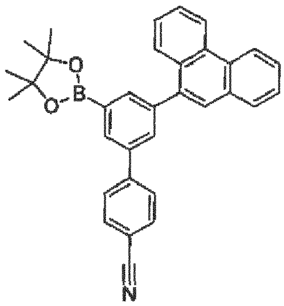

Under nitrogen, a flask was charged with 4,6g from intermediate 3'-chloro-5'-(phenanthren-9-yl)-[1,1'-biphenyl]-4-carbonitrile, in 120mL dry dioxane. Under counterflow of nitrogen were added bis(pinacolato)diboron (3,9g 1,3eq.), 2,5%mol of catalyst Pd2(dba)3, 5%mol of XPhos ligand and finally 3eq. of potassium acetate. The reaction mixture was heated to 110°C with stirring for 24h. The mixture was allowed to cool down to the room temperature, dioxane was evaporated. Extraction with 0,5L dichloromethane was performed. After washing the organic layer with 3x50mL water, it was dried over MgSO4 and filtered over florisil. Further purification was achieved by silicagel filtration (Dichloromethane/Cyclohexane 1:1) to afford 4,2g (73%) product, m/z 504 $[M+Na]^+$

Example of reaction conditions which can be suitably used for preparation of compound of Formula (I) from compound of Formula 2

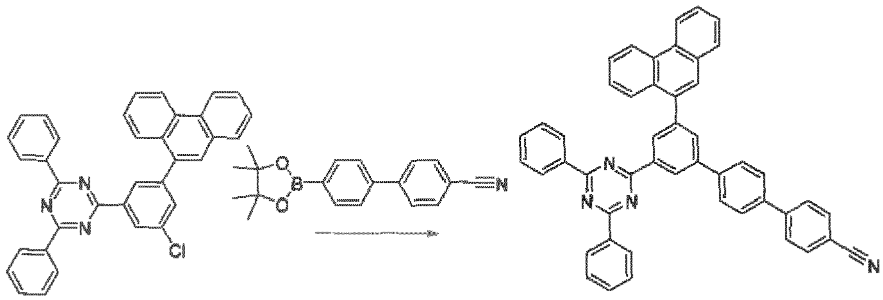

Potassium phosphate K3PO4 (as 10m L aqueous solution, 2 eq.) was degassed with nitrogen for 30 min. THF (41 ml) was also degassed with nitrogen for 30 min. The THF flask was then charged with previously synthesized intermediate of formula 2 (4.5 g, 1eq.), 4'-(4,4,5,5-tetramethyl-1,3,2-dioxaborolan-2-yl)-[1,1'-biphenyl]-4-carbonitrile [406482-72-2] (1.1eq.), and palladium source Pd172 [CAS 1798781-99-3] (0.02 eq.) under a positive nitrogen pressure. The degassed potassium phosphate solution was added, nitrogen purged reflux condenser was attached to the flask and a reaction mixture heated to 45 °C with stirring for 18 h. The mixture was allowed to cool down to the room temperature. The precipitate was filtered, washed thoroughly with water. After dissolution in refluxing chlorobenzene and filtration on silicagel layer, the reduction of the solvent afforded white powder that was recrystallized in 100mL chlorobenzene to afford 4.7g (82%) product, m/z 663 $[M+H]^+$

Compounds of formula (I) below were prepared as described above:

| | Starting compound(s), | Product | Yield and MS data |
|---|---|---|---|
| A-3 | 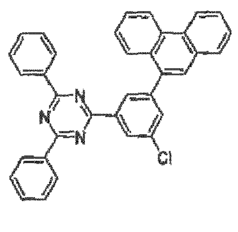 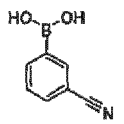 | 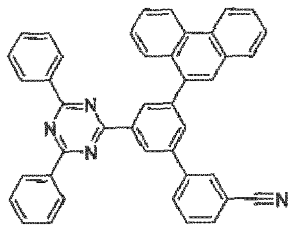 | 76% 587$[M+H]^+$ |
| A-2 | 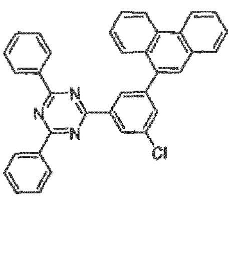 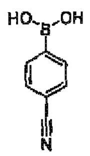 | 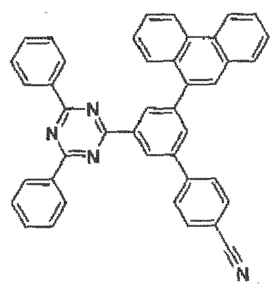 | 58% 587$[M+H]^+$ |

(continued)

| | Starting compound(s), | Product | Yield and MS data |
|---|---|---|---|
| A-4 | 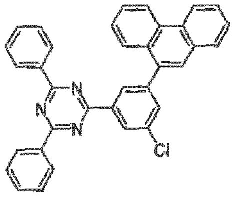 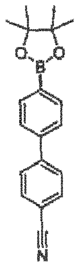 | 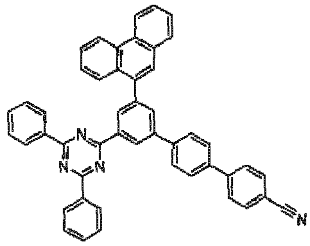 | 82% 663[M+H]+ |
| A-17 | 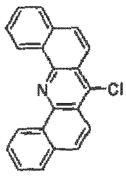 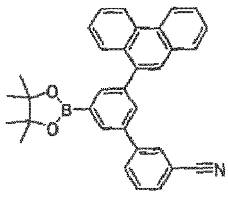 | 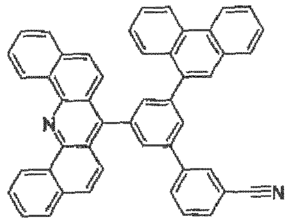 | 41% 633[M+H]+ |
| A-18 | 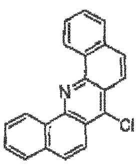 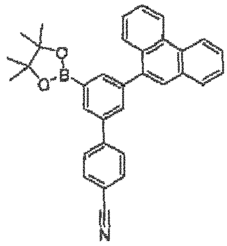 | 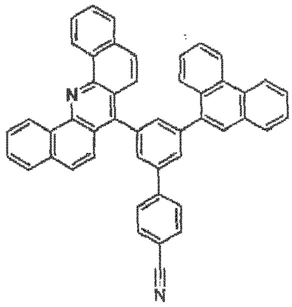 | 49% 663[M+H]+ |
| A-9 | 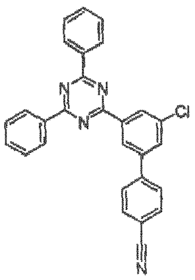 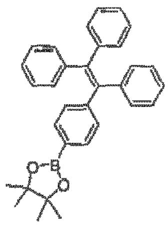 | 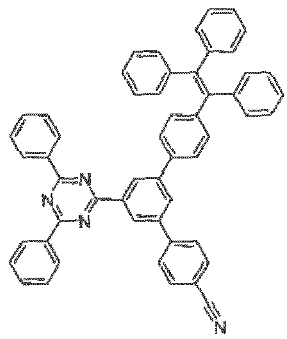 | 63% 741[M+H] + |

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

The melting point of a compound of Formula (I) may be selected in the range of about 250 to about 380 °C, preferably about 260 to about 370 °C, also preferred about 270 to about 360 °C.

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

The glass transition temperature of a compound of Formula (I) may be selected in the range of about 115 to about 200 °C, preferably about 120 to about 190 °C, also preferred about 125 to about 180 °C.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behaviour.

Rate onset temperature

The rate onset temperature ($T_{RO}$) is determined by loading 100 mg compound into a VTE source. The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

The rate onset temperature of a compound of Formula (I) may be selected in the range of about 230 to about 290 °C, preferably about 235 to about 280 °C.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

The dipole moment of a compound of Formula (I) may be selected in the range of about 3 to about 6 Debye, preferably about 3.2 to about 6 Debye, also preferred about 3.4 to about 6 Debye.

HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

The HOMO of a compound of Formula (I) may be selected in the range of about -5.2 to about - 5.9 eV, preferably about -5.3 to about -5.85 eV, also preferred about -5.4 to about -5.85 eV.

The LUMO of a compound of Formula (I) may be selected in the range of about -1.8 to about - 2.1 eV, preferably about -1.85 to about -2.08 eV, also preferred about -1.85 to about -2.06 eV.

General procedure for fabrication of OLEDs

For top emission OLED devices, Examples 1 to 3 and comparative example 1, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare the substrate. 100 nm Ag were deposited on the substrate at a pressure of $10^{-5}$ to $10^{-7}$ mbar to form an anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the anode, to form a hole injection layer (HIL) having a thickness of 10 nm. Then, Biphenyl-4-yl(9,9-diphenyl-9H-fluoren-2-yl)-[4-(9-phenyl-9H-carbazol-3-yl)phenyl]-amine was vacuum deposited on the HIL, to form a hole transport layer (HTL) having a thickness of 118 nm.

Then, N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then, the emission layer (EML) was deposited. 97 vol.-% H09 (Sun Fine Chemicals) as EML host and 3 vol.-% BD200 (Sun Fine Chemicals) as fluorescent blue dopant were deposited on the EBL, to form a blue-emitting EML with a thickness of 20 nm.

Then, 2,4-diphenyl-6-(4',5',6'-triphenyl-[1(1':2',1":3",1"':3"',1""-quinquephenyl]-3""-yl)-1,3,5-triazine was deposited on the EML, to form a hole blocking layer (HBL) with a thickness of 5 nm.

Then, the electron transport layer (ETL) is formed on the hole blocking layer with a thickness of 31 nm by co-deposition of a matrix compound and an alkali organic complex. The composition of the ETL is shown in Table 2.

Then, the electron injection layer (EIL) is formed on the electron transporting layer by deposing Yb with a thickness of 2 nm.

Ag and Mg in a ratio of 90:10 vol.-% are evaporated at a rate of 0.01 to 1 Å/s at $10^{-7}$ mbar to form a cathode with a thickness of 13 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 70 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristics are determined using a Keithley 2635 source measure unit by sourcing a voltage in V and measuring the current in mA flowing through the device under test. The voltage applied to the device is varied in steps of 0.1V in the range between oV and 10V. Likewise, the luminance-voltage characteristics and CIE coordinates are determined by measuring the luminance in cd/$m^2$ using an Instrument Systems CAS-140CT array spectrometer, which has been calibrated by Deutsche Akkreditierungsstelle (DAkkS) for each of the voltage values. The cd/A efficiency at 10 mA/$cm^2$ is determined by interpolating the luminance-voltage and current-voltage characteristics, respectively.

Lifetime LT of the device is measured at ambient conditions (20°C) and 30 mA/$cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

## Technical Effect of the invention

Properties of compounds of Formula (I)

In Table 1 are shown mp, $T_g$, $T_{RO}$, HOMO and LUMO energy levels and the dipole moments of compounds of Formula (I) and of comparative compounds 1 to 4. Surprisingly, compounds of Formula (I) have improved melting points, increased glass transition temperature and increased dipole moments compared compounds known in the art comprising phenyl or pyridyl groups. Additionally, the $T_{RO}$ is in the range suitable for mass production.

In Table 2 are shown the composition of the organic semiconducting layer, operating voltage, cd/A efficiency and lifetime. In comparative example 1, the organic semiconducting layer comprises a compound known in the art as matrix compound and LiQ as dopant. In Examples 1 to 3, the organic semiconducting layer comprises a compound of Formula (I) as matrix compound and LiQ as dopant. As can be seen in Table 2, higher cd/A efficiency and/or improved lifetime are obtained. Improved cd/A efficiency may result in reduced power consumption and thereby in improved battery life in mobile displays. Improved lifetime is important for the long-term stability of a device.

In summary, compounds of Formula (I) and organic electronic devices comprising an organic semiconducting layer comprising of compound of Formula (I) show superior performance over the state of the art.

Table 1: Properties of comparative compounds 1 to 4 and compounds of Formula (I)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| Comparative compound 1 | 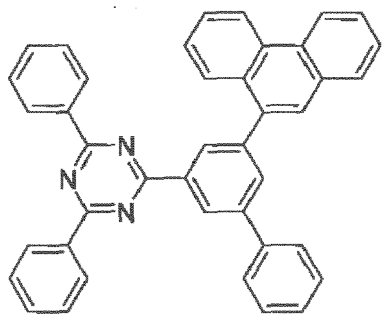 | - | - | - | -5.64 | -1.84 | 0.13 |
| Comparative compound 2 | 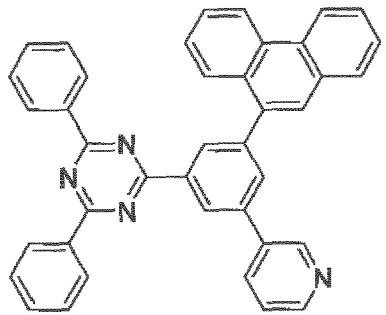 | 250 | 115 | 218 | -5.70 | -1.93 | 2.51 |
| Comparative compound 3 | 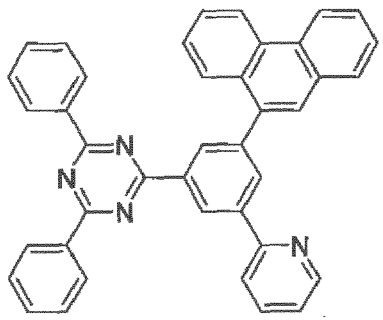 | 242 | - | 226 | -5.59 | -1.85 | 1.77 |

(continued)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| Comparative compound 4 | 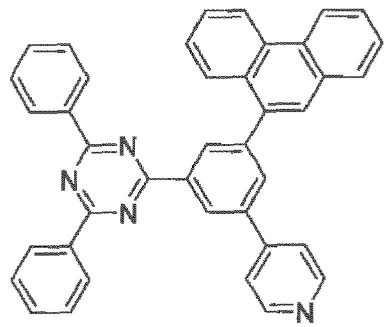 | 248 | - | 255 | -5.76 | -1.97 | 3.06 |
| A-3 | 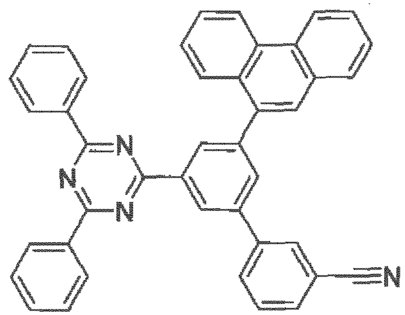 | 276 | 120 | 238 | -5.77 | -2.02 | 5.04 |
| A-2 | 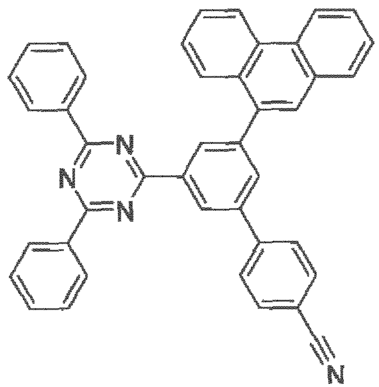 | 291 | 133 | 246 | -5.83 | -2.06 | 5.68 |

(continued)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| A-4 | 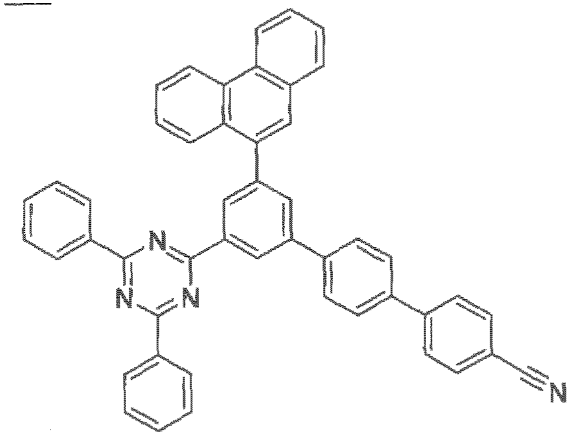 | - | - | - | -5.77 | -1.99 | 5.85 |
| A-17 | 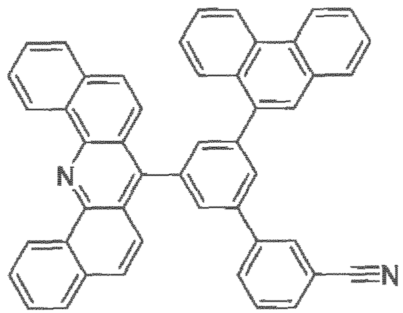 | 282 | 145 | 266 | -5.76 | -1.88 | 3.44 |

(continued)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| A-18 | 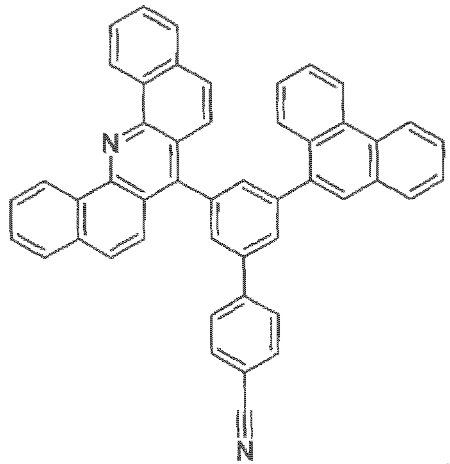 | 292 | 168 | 270 | -5.77 | -1.89 | 4.99 |
| A-9 | 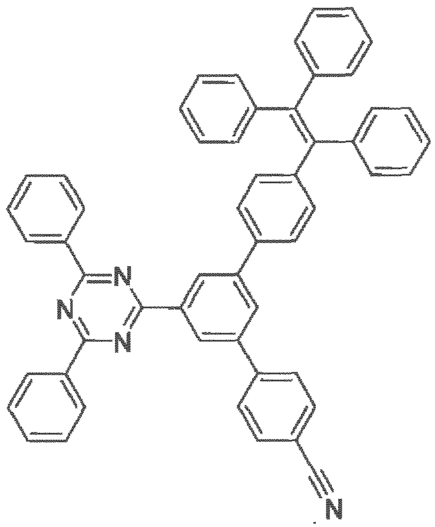 | 316 | 145 | 269 | -5.42 | -2.03 | 5.89 |

Table 2: Performance of an organic electroluminescent device comprising an electron transport layer comprising a matrix compound and an alkali metal complex.

| | Matrix compound | Concentration of matrix compound (vol.-%) in the ETL | Alkali metal complex | Concentration of alkali metal complex (vol.-%) in the ETL | Thickness of the ETL (nm) | Operating voltage at 10 mA/cm$^2$ (V) | cd/A efficiency at 10 mA/cm$^2$ (cd/A) | LT97 at 30 mA/cm$^2$ (h) |
|---|---|---|---|---|---|---|---|---|
| Comparative example 1 | Comparative compound 2 | 50 | LiQ | 50 | 31 | 3.7 | 7.4 | 226 |
| Example 1 | A-3 | 50 | LiQ | 50 | 31 | 3.7 | 7.5 | 258 |
| Example 2 | A-2 | 50 | LiQ | 50 | 31 | 3.7 | 7.3 | 277 |
| Example 3 | A-9 | 50 | LiQ | 50 | 31 | 3.6 | 7.6 | 246 |

The features disclosed in the foregoing description and in the dependent claims may, both separately and in any combination thereof, be material for realizing the aspects of the disclosure made in the independent claims, in diverse forms thereof.

## Claims

1. Compound of the Formula (I)

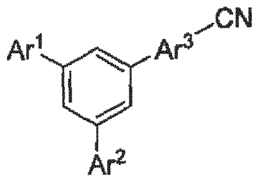

(I)

wherein $Ar^1$ is selected from substituted or unsubstituted $C_2$ to $C_{36}$ heteroaryl and the group $Ar^1$ is bound to the remaining structure represented by Formula (I) via one of its carbon atoms;
$Ar^2$ is selected from the group consisting of substituted or unsubstituted $C_6$ to $C_{36}$ aryl and substituted or unsubstituted $C_4$ to $C_{36}$ heteroaryl comprising at least one heteroatom selected from O, S and Se, wherein the heteroaryl comprising at least one heteroatom selected from O, S and Se is bound to the remaining structure represented by Formula (I) via one of its carbon atoms;
$Ar^3$ is selected from the group consisting of substituted or unsubstituted $C_6$ to $C_{18}$ arylene and substituted or unsubstituted $C_3$ to $C_{18}$ heteroarylene, wherein it is provided that the group $Ar^3$ comprises not more than three fused rings;
wherein, in case that one or more of $Ar^1$, $Ar^2$ and $Ar^3$ is substituted, the substituents are each independently selected from the group consisting of D, F, $C_1$ to $C_{20}$ linear alkyl, $C_3$ to $C_{20}$ branched alkyl, $C_3$ to $C_{20}$ cyclic alkyl, $C_1$ to $C_{20}$ linear alkoxy, $C_3$ to $C_{20}$ branched alkoxy, linear fluorinated $C_1$ to $C_{12}$ alkyl, linear fluorinated $C_1$ to $C_{12}$ alkoxy, $C_3$ to $C_{12}$ branched fluorinated cyclic alkyl, $C_3$ to $C_{12}$ fluorinated cyclic alkyl, $C_3$ to $C_{12}$ fluorinated cyclic alkoxy, CN, RCN, $C_6$ to $C_{20}$ aryl, $C_2$ to $C_{20}$ heteroaryl, OR, SR, (C=O)R, (C=O)$NR_2$, $SiR_3$, (S=O)R, $(S=O)_2R$, $(P=O)R_2$; wherein each R is independently selected from $C_1$ to $C_{20}$ linear alkyl, $C_1$ to $C_{20}$ alkoxy, $C_1$ to $C_{20}$ thioalkyl, $C_3$ to $C_{20}$ branched alkyl, $C_3$ to $C_{20}$ cyclic alkyl, $C_3$ to $C_{20}$ branched alkoxy, $C_3$ to $C_{20}$ cyclic alkoxy, $C_3$ to $C_{20}$ branched thioalkyl, $C_3$ to $C_{20}$ cyclic thioalkyl, $C_6$ to $C_{20}$ aryl and $C_2$ to $C_{20}$ heteroaryl; and wherein $Ar^1$ and $Ar^2$ are selected differently from each other.

2. Compound according to claim 1, wherein $Ar^1$ is selected from $C_3$ to $C_{26}$ heteroaryl.

3. Compound according to claim 1 or 2, wherein $Ar^1$ comprises at least one N atom.

4. Compound according to any of the preceding claims, wherein $Ar^2$ comprises at least two fused rings.

5. Compound according to any of the claims 1 to 3, wherein $Ar^2$ is selected from groups having the following formulas a to k

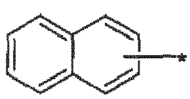

a

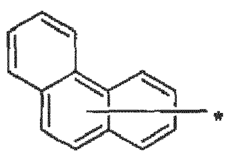

b

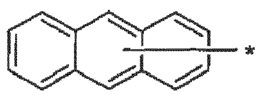

c

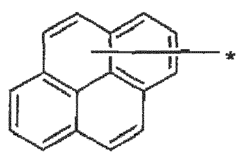

d

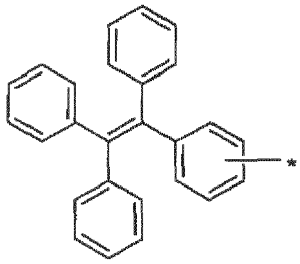 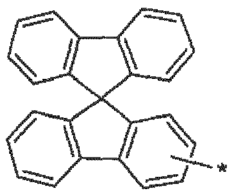 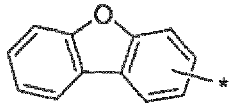

e f g

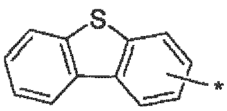 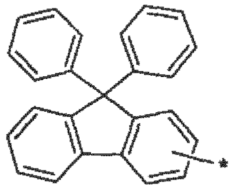 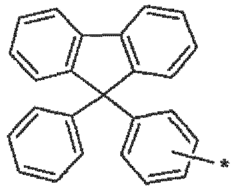 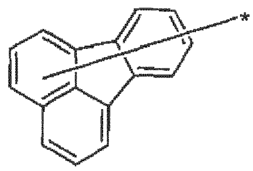

h i j k.

6. Compound according to any of the preceding claims, wherein $Ar^3$ is selected from the group consisting of substituted or unsubstituted $C_6$ to $C_{12}$ arylene and substituted or unsubstituted $C_5$ to $C_{16}$ heteroarylene.

7. Compound according to any of the preceding claims, wherein $Ar^1$ is selected from formulas 1 to dd

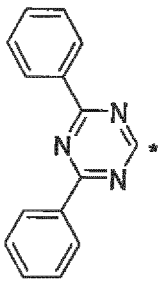 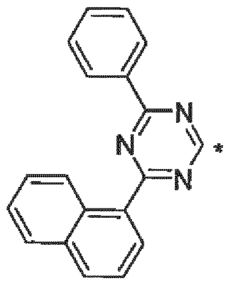 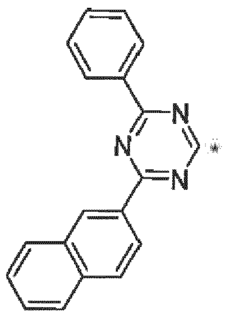 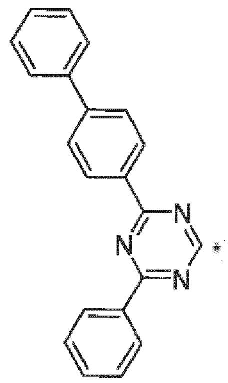

l m n o

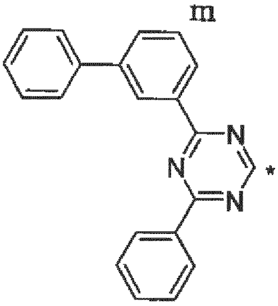 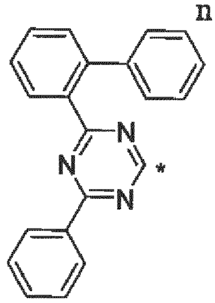 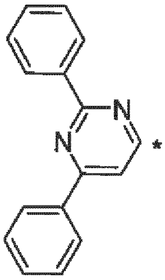 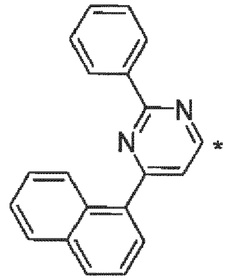

p q r s

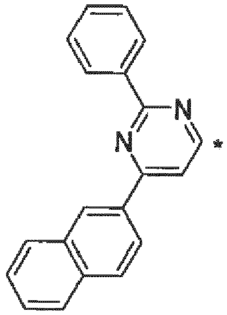
t

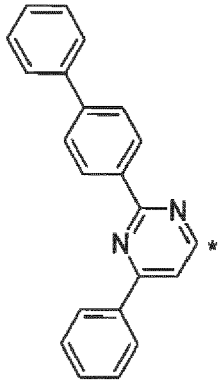
u

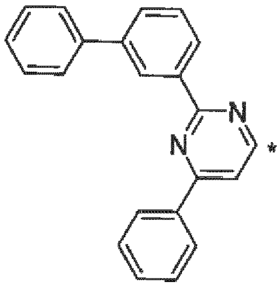
v

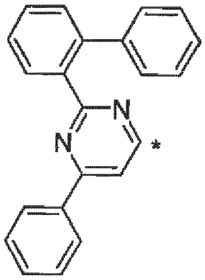
w

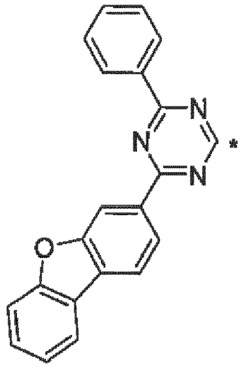
x

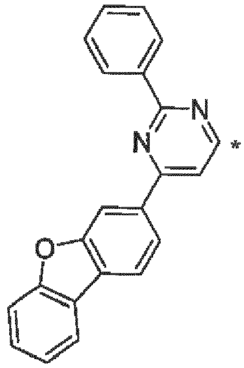
y

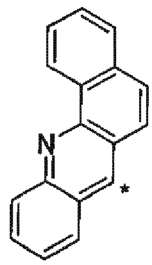
z

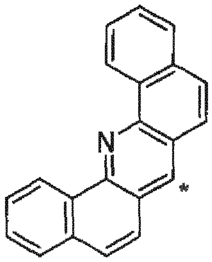
aa

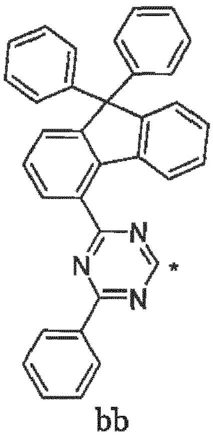
bb

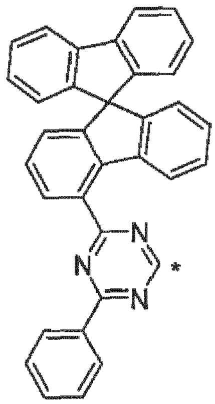
cc

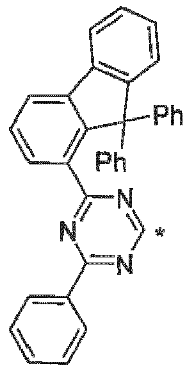
dd

wherein the asterisk symbol "*" in the above formulas represents the position of binding of the respective group to the remaining structure represented by Formula (I).

**8.** Compound according to any of the preceding claims, wherein the compound is selected from the following structures A-1 to A-18

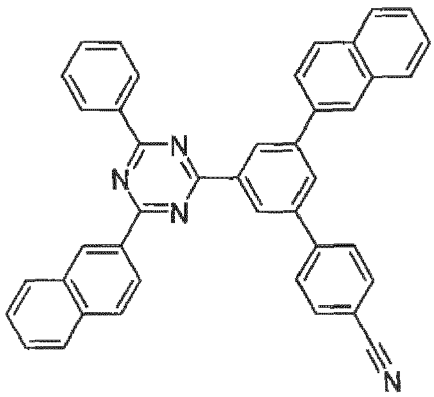

A-1

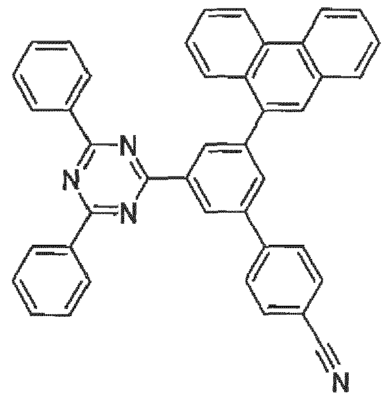

A-2

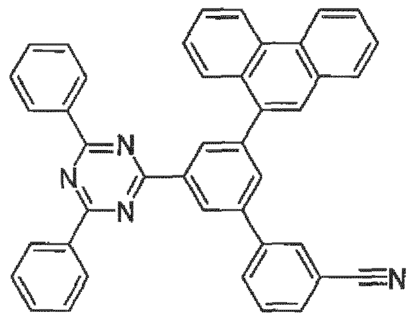

A-3

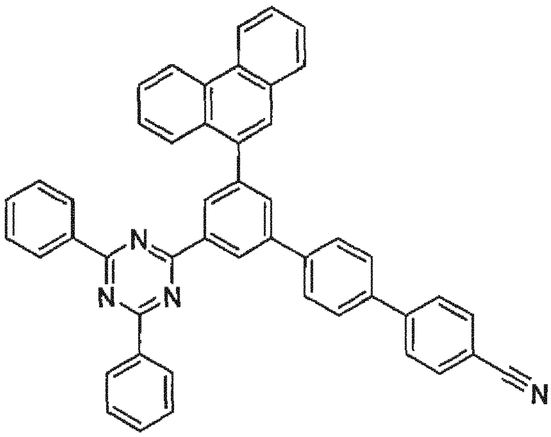

A-4

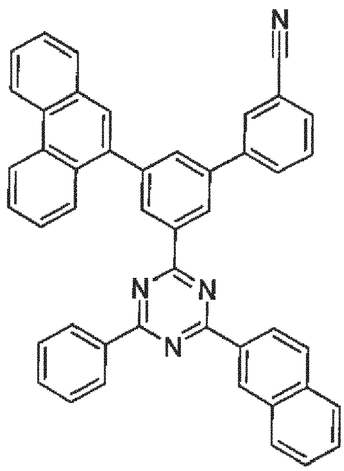

A-5

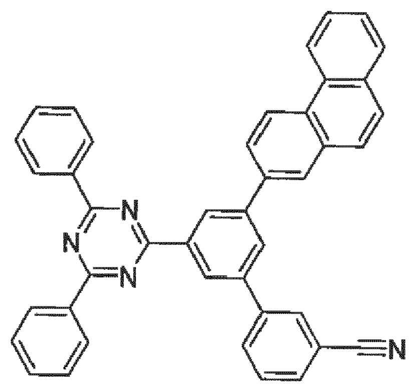

A-6

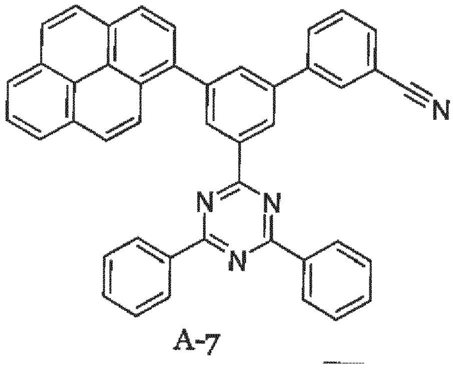

A-7

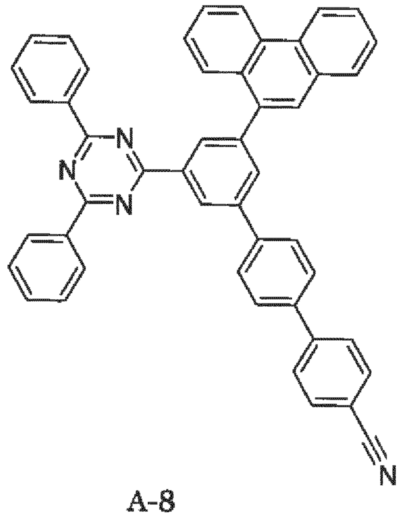

A-8

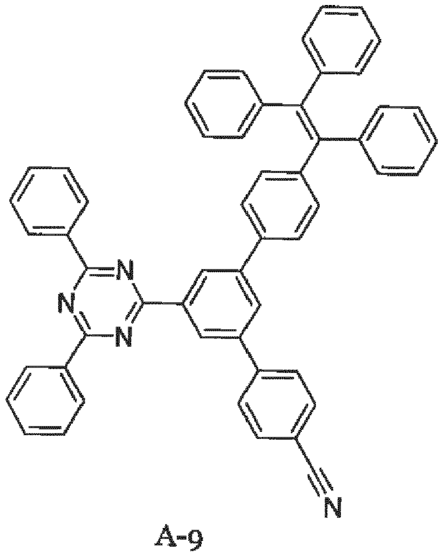

A-9

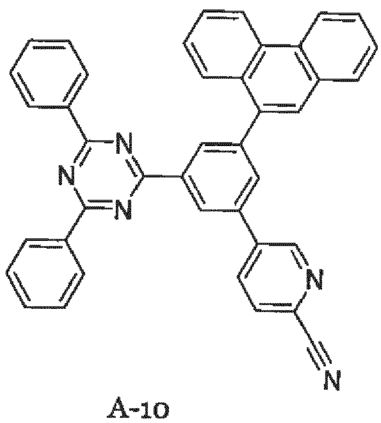

A-10

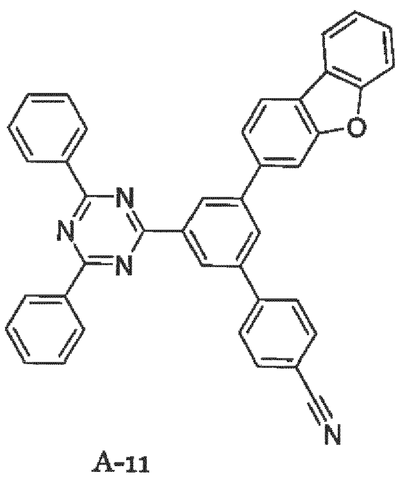

A-11

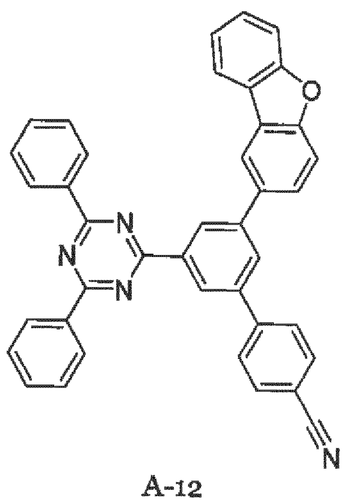

A-12

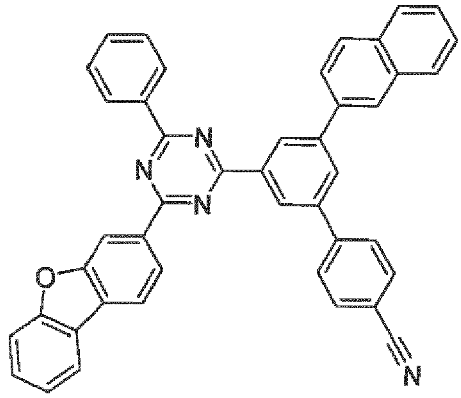

A-13

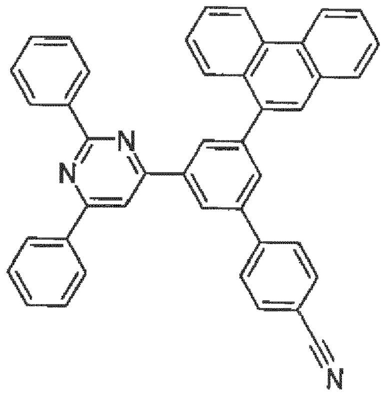

A-14

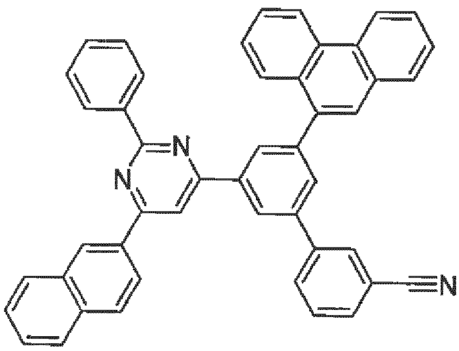

A-15

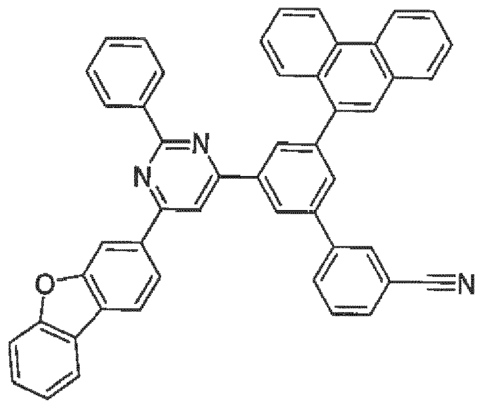

A-16

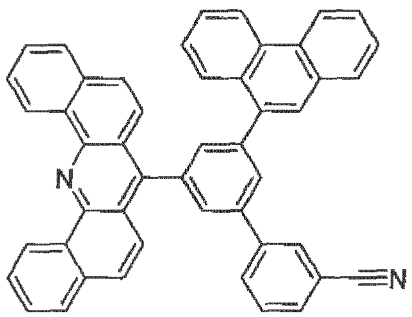

A-17

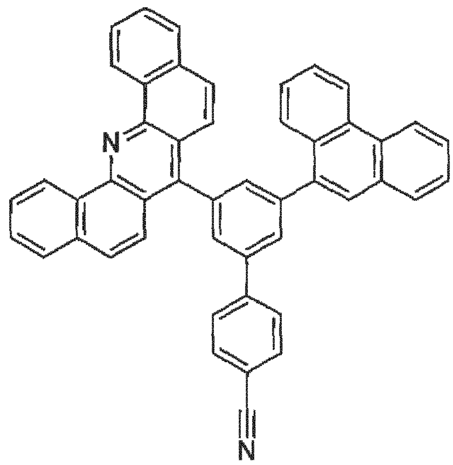

A-18

**9.** Organic semiconducting layer comprising at least one compound of Formula (I) according to any of the preceding claims.

**10.** Organic semiconducting layer according to claim 9, wherein the organic semiconducting layer further comprises a metal, a metal salt or an alkali or alkaline earth metal complex, alternatively an organic alkali or alkaline earth metal complex.

**11.** Organic electronic device comprising the organic semiconducting layer according to any of the claims 9 or 10.

**12.** Organic electronic device according to claim 11 further comprising an emission layer and a cathode, wherein the organic semiconducting layer is arranged between the emission layer and the cathode.

**13.** Display device comprising the organic electronic device according to any of the claims 11 or 12.

**14.** Process for preparing a compound of Formula (I) according to any of the claims 1 to 8 comprising the steps of

(a) providing a compound of Formula 2 or 3

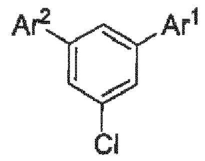

Formula 2

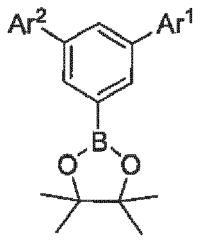

Formula 3;

(b) reacting the compound of Formula 2 with a compound having the formula NC-$Ar^3$-$B(OR)_2$, wherein R is selected from H, $C_1$ to $C_4$ alkyl or

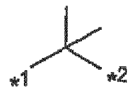

,

wherein the asterisk symbol "$*^1$" in the above formula represents the position of binding of the respective group to the O and the asterisk symbol "$*^2$" in above formula represents the position of binding of the respective group to the other R;
or reacting the compound of Formula 3 with a compound having the formula NC-$Ar^3$-Y, wherein Y is selected from the group consisting of I, Br, Cl and OTf.

**15.** Process for preparing a compound of Formula (I) according to any of the claims 1 to 8 comprising the steps of

(a) providing a compound of Formula 7

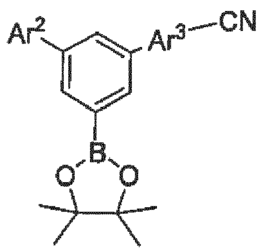

Formula 7;

(b) reacting the compound of Formula 7 with a compound having the formula $Ar^1$-Y, wherein Y is selected from the group consisting of I, Br, Cl and OTf.

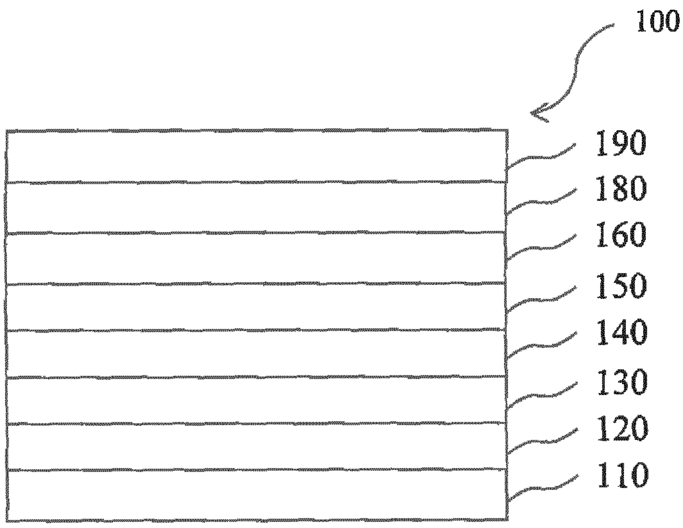
100
190
180
160
150
140
130
120
110

Fig.1

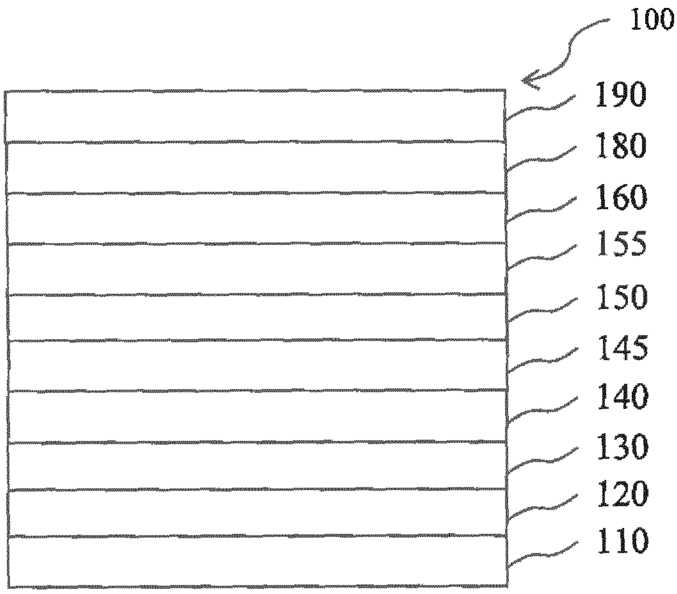
100
190
180
160
155
150
145
140
130
120
110

Fig.2

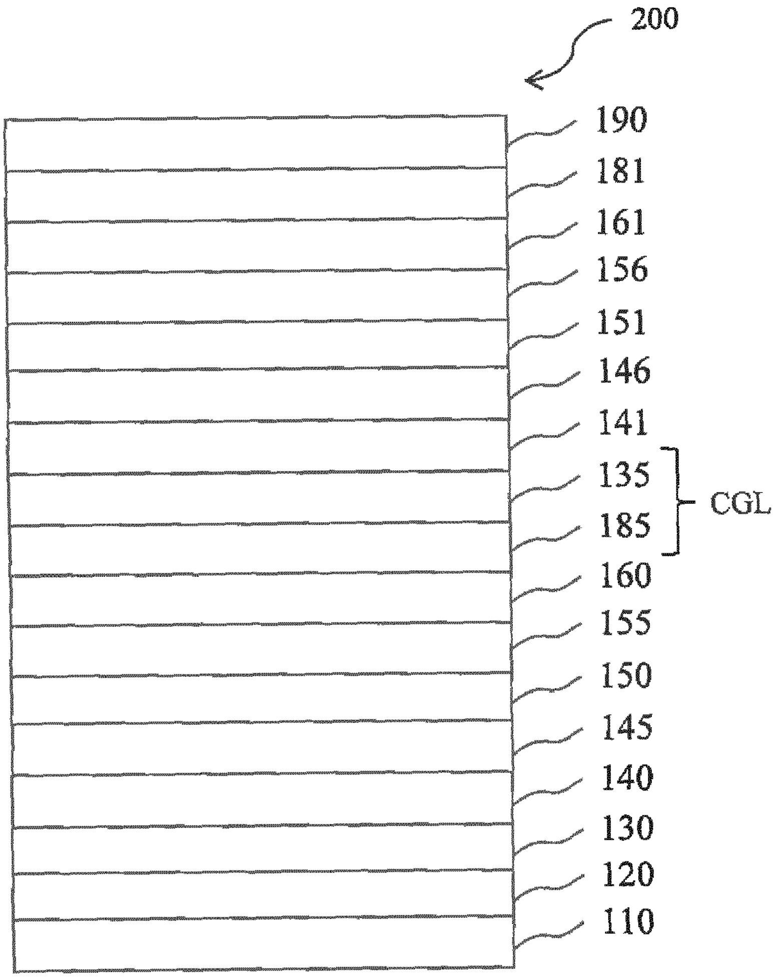
200
190
181
161
156
151
146
141
135
185
CGL
160
155
150
145
140
130
120
110

Fig.3

Europäisches Patentamt
European Patent Office
Office européen des brevets

# EUROPEAN SEARCH REPORT

Application Number
EP 18 16 8029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2017 0058625 A (LAPTO CO LTD [KR]) 29 May 2017 (2017-05-29) * the whole document; in particular the claims and compounds 7-35, 7-36 * | 1-15 | INV. C07D221/18 C07D401/10 C07D239/26 C07D405/10 C07D251/24 H01L51/00 H01L51/50 |
| X<br>Y | KR 2017 0090139 A (LAPTO CO LTD [KR]) 7 August 2017 (2017-08-07) * the whole document; in particular the claims and compounds 4-16, 4-18, 4-28, 4-41, 4-50, 4-53, 4-54, 4-61, 4-109, 4-121, 4-136, 4-145, 4-148, 4-149, 4-156 * | 1-4,6,7, 9-15<br>5,8 | |
| X<br>Y | US 2017/054082 A1 (KIM HYUNJUNG [KR] ET AL) 23 February 2017 (2017-02-23) * the whole document; in particular the claims and compounds 4-9, 13-18, 22-27, 31-36, 40-45, 49-54, 58-63, 67-72, 76-81 * | 1,2,6, 9-15<br>3-5,7,8 | |
| X<br>Y | US 2014/203251 A1 (JUNG JOON HO [KR] ET AL) 24 July 2014 (2014-07-24) * the whole document; in particular the claims and compound 4-4 * | 1,2,6, 9-13<br>3-5,7,8 | TECHNICAL FIELDS SEARCHED (IPC) |
| X<br>Y | KR 2016 0083184 A (LG DISPLAY CO LTD [KR]) 12 July 2016 (2016-07-12) * the whole document; in particular the claims and the following compounds: the last compound in [0085], all compounds in [0086], [0088] and [0090] and the first and the last compound in [0091] on pages 17-18 * | 1-3,6, 9-15<br>4,5,7,8 | C07D |
| X<br>Y | KR 2016 0122432 A (LMS CO LTD [KR]) 24 October 2016 (2016-10-24) * the whole document; in particular the claims and compound a-16 * | 1-3,6, 9-15<br>4,5,7,8 | |

-/--

The present search report has been drawn up for all claims

1

| Place of search | Date of completion of the search | Examiner |
|---|---|---|
| Munich | 20 September 2018 | Hanisch, Inken |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 16 8029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2017/098780 A1 (KIM HYUNJUNG [KR] ET AL) 6 April 2017 (2017-04-06)<br>* the whole document; in particular the claims and compounds 22, 23, 60, 61, 68, 69, 102 and 103 *<br>----- | 1,2,6, 9-15<br>3-5,7,8 | |

TECHNICAL FIELDS SEARCHED (IPC)

The present search report has been drawn up for all claims

1

| Place of search | Date of completion of the search | Examiner |
|---|---|---|
| Munich | 20 September 2018 | Hanisch, Inken |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

page 2 of 2

## ANNEX TO THE EUROPEAN SEARCH REPORT ON EUROPEAN PATENT APPLICATION NO. EP 18 16 8029

This annex lists the patent family members relating to the patent documents cited in the above-mentioned European search report. The members are as contained in the European Patent Office EDP file on The European Patent Office is in no way liable for these particulars which are merely given for the purpose of information.

20-09-2018

| Patent document cited in search report | Publication date | Patent family member(s) | Publication date |
|---|---|---|---|
| KR 20170058625 A | 29-05-2017 | NONE | |
| KR 20170090139 A | 07-08-2017 | NONE | |
| US 2017054082 A1 | 23-02-2017 | KR 20170022411 A<br>US 2017054082 A1 | 02-03-2017<br>23-02-2017 |
| US 2014203251 A1 | 24-07-2014 | KR 20120132423 A<br>US 2014203251 A1<br>WO 2012161554 A2 | 05-12-2012<br>24-07-2014<br>29-11-2012 |
| KR 20160083184 A | 12-07-2016 | NONE | |
| KR 20160122432 A | 24-10-2016 | NONE | |
| US 2017098780 A1 | 06-04-2017 | KR 20170040697 A<br>US 2017098780 A1 | 13-04-2017<br>06-04-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1
- EP 1970371 A1
- WO 2013079217 A1


### Non-patent literature cited in the description


- **YASUHIKO SHIROTA ; HIROSHI KAGEYAMA.** *Chem. Rev.,* 2007, vol. 107, 953-1010
- *CHEMICAL ABSTRACTS,* 1798781-99-3
- *CHEMICAL ABSTRACTS,* 1242056-42-3
- *CHEMICAL ABSTRACTS,* 1198399-61-9